# EUROPÄISCHE PATENTSCHRIFT

(11) **EP 1 603 888 B1**
(45) Veröffentlichungstag und Bekanntmachung des Hinweises auf die Patenterteilung: **12.01.2011**
(21) Anmeldenummer: 04706148.6
(22) Anmeldetag: 29.01.2004
(51) Int. Cl.: C07D 239/84, A61K 31/517, A61P 31/22

(54) **2-(3-PHENYL-2-PIPERAZINYL-3,4-DIHYDROCHINAZOLIN-4-YL) ESSIGSÄUREN ALS ANTIVIRALE MITTEL, SPEZIELL GEGEN CYTOMEGALIEVIREN**
2-(3-PHENYL-2-PIPERAZINYL-3,4-DIHYDROQUINAZOLINE-4-YL) ACETIC ACIDS AS ANTI-VIRAL AGENTS, ESPECIALLY AGAINST CYTOMEGALO VIRUSES
ACIDES 2-(3-PHENYL-2-PIPERAZINYL-3,4-DIHYDROCHINAZOLINO-4-YL)-ACETIQUES SERVANT D'AGENTS ANTIVIRAUX, NOTAMMENT CONTRE DES CYTOMEGALOVIRUS

(30) Priorität: 12.02.2003 DE 10305785
(43) Veröffentlichungstag der Anmeldung: 14.12.2005
(73) Patentinhaber: Bayer Schering Pharma Aktiengesellschaft, 13353 Berlin (DE)
(72) Erfinder: WUNBERG, Tobias, 42699 Solingen (DE); BAUMEISTER, Judith, 2800 Mechelen (BE); BETZ, Ulrich, 64354 Reinheim (DE); JESKE, Mario, 42699 Solingen (DE); KLEYMANN, Gerald, 32107 Bad Salzuflen (DE); LAMPE, Thomas, 40545 Düsseldorf (DE); NIKOLIC, Susanne, 40789 Monheim (DE); REEFSCHLÄGER, Jürgen, 26125 Odenthal (DE); SCHOHE-LOOP, Rudolf, 42327 Wuppertal (DE); SÜßMEIER, Frank, 42275 Wuppertal (DE); ZIMMERMANN, Holger, 42113 Wuppertal (DE); GROSSER, Rolf, 51373 Leverkusen (DE); HENNINGER, Kerstin, 42115 Wuppertal (DE); HEWLETT, Guy, 42327 Wuppertal (DE); KELDENICH, Jörg, 42113 Wuppertal (DE); KRÄMER, Thomas, 42111 Wuppertal (DE); NELL, Peter, 42115 Wuppertal (DE); LIN, Tse-I, 2800 Mechelen (BE)
(86) Internationale Anmeldenummer: PCT/EP2004/000783
(87) Internationale Veröffentlichungsnummer: WO 2004/072048

(56) Entgegenhaltungen:
- WO-A-99/41253
- DE-A- 4 320 347
- WANG, FENGJLANG ET AL: "Solid-phase synthesis of 3,4-dihydroquinazoline" TETRAHEDRON LETTERS (1997), 38(50), 8651-8654 , XP004097142 in der Anmeldung erwähnt
- SAITO, TAKAO ET AL: "A facile and efficient carbodiimide-mediated synthesis of dihydroquinazolines via a tandem nucleophilic addition-intramolecular hetero conjugate addition annulation strategy" TETRAHEDRON LETTERS (1996), 37(2), 209-12 , XP004030439 in der Anmeldung erwähnt

## Beschreibung

Die Erfindung betrifft Dihydrochinazoline und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

Die Synthese von Dihydrochinazolinen ist beschrieben in Saito T., et al. Tetrahedron Lett., 1996, 37,209-212 und in Wang F., et al. Tetrahedron Lett., 1997, 38, 8651-8654.

Auf dem Markt sind zwar strukturell andersartige antiviral wirkende Mittel vorhanden, aber die gegenwärtig verfügbaren Therapien mit Ganciclovir, Valganciclovir, Foscarnet und Cidofovir sind mit schweren Nebenwirkungen verbunden, z.B. Nephrotoxizität, Neutropenie oder Thrombozytopenie. Zudem kann es regelmäßig zu einer Resistenzentwicklung kommen. "Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infeh-tionskrankheiten bei Menschen und Tieren zur Verfügung zu stellern.

Überraschenderweise wurde gefunden, dass die in der vorliegenden Erfindung beschriebenen Dihydrochinazoline antiviral wirksam sind.

Gegenstand der Erfindung sind Verbindungen der Formel 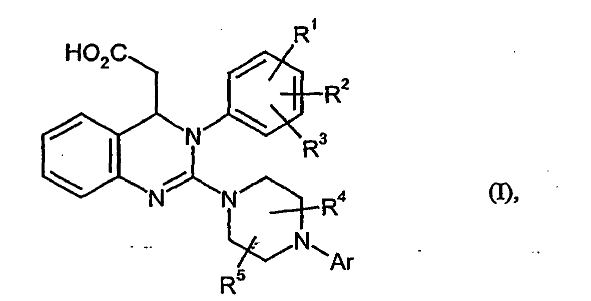 in welcher
- Ar: für C₆-C₁₄-Aryl steht, worin Aryl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Formyl, Carboxyl, C₁-C₆-Alkylcarbony, C₁-C₆-Alkoxycarbonyl, Trifluormethyl, Halo- gen, Cyano, Hydroxy, Amino, C₁-C₆-Alkylamino, Aminocarbonyl und Nitro, worin Alkyl substituiert sein kann mit 1 bis 3 Substituenten, wobei die Substituenten unab- hängig voneinander ausgewählt werden aus der Gruppe, bestehend aus Halogen, Amino, C₁-C₆-Alkyl amino, Hydroxy und C₆-C₁₄-Aryl, oder zwei der Substituenten am Aryl zusammen mit den Kohlenstoffatomen, an die sie gebunden sind, ein 1,3-Dioxolan, einen Cyclopentan-Ring oder einen Cyclohexan-Ring bilden und ein gegebenenfalls vorhandener dritter Substituent unabhängig davon aus der genannten Gruppe ausgewählt wird, R¹ für Wasserstoff, C₁-C₆-Alkyl, C₁-C₆-Alkoxy, Cyano, Halogen, Nitro oder Trifluormethyl steht,
- R²: für Wasserstoff, C₁-C₆-Alkyl, C₁-C₆-Alkoxy, Cyano, Halogen, Nitro oder Trifluormethyl steht,
- R³: für C₁-C₆-Alkyl, C₁-C₆-Alkoxy, Cyano, Halogen, Nitro oder Trifluormethyl steht
oder
einer der Reste R¹, R² und R³ für Wasserstoff, C₁-C₆-Alkyl, C₁-C₆-Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,
- R⁴: für Wasserstoff oder C₁-C₆-Alkyl steht
und
- R⁵: für Wasserstoff oder C₁-C₆-Alkyl steht
oder
die Reste R⁴ und R⁵ im Piperazin-Ring an genau gegenüberliegenden Kohlenstoffatomen gebunden sind und eine gegebenenfalls mit 1 bis 2 Methylgruppen substituierte Methylen-Brücke bilden,
und ihre Salze, ihre Solvate und die Solvate ihrer Salze.

Erfindungsgemäße Verbindungen sind die Verbindungen der Formel (I) und deren Salze, Solvate und Solvate der Salze, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylcarbonyl und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.
Alkoxy steht beispielhaft und vorzugsweise für Methoxy, Ethoxy, n-Propoxy, Isopropoxy, tert.-Butoxy, n-Pentoxy und n-Hexoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N*-Dimethylamino, *N,N-*Diethylamino, *N-*Ethyl-N-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
Alkylcarbonyl steht beispielhaft und vorzugsweise für Acetyl und Propanoyl.
Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.
Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.
Halogen steht für Fluor, Chlor, Brom und Jod, bevorzugt Fluor und Chlor.
Ein Symbol * an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90 % verstanden wird (> 90 % ee).

Bevorzugt sind solche Verbindungen der Formel (I), in welchen
- Ar: für Phenyl steht, worin Phenyl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Carboxyl, C₁-C₆-Alkylcarbonyl, C₁-C₆-Alkoxycarbonyl, Tri- fluormethyl, Fluor, Chlor, Brom, Cyano, Hydroxy, Amino, C₁-C₆-Alkylamino und Nitro,
oder zwei der Substituenten am Aryl zusammen mit den Kohlenstoffatomen, an die sie ge- bunden sind, ein 1,3-Dioxolan bilden und ein gegebenenfalls vorhandener dritter Substituent unabhängig davon aus der genannten Gruppe ausgewählt wird,
- R¹: für Wasserstoff, C₁-C₃-Alkyl, C₁-C₃-Alkoxy, Fluor oder Chlor steht,
- R²: für Wasserstoff, C₁-C₃-Alkyl, C₁-C₃-Alkoxy, Fluor oder Chlor steht,
- R³: für C₁-C₄-Alkyl, Cyano, Fluor, Chlor, Nitro oder Trifluormethyl steht,
- R⁴: für Wasserstoff oder Methyl steht
und
- R⁵: für Wasserstoff steht,
und ihre Salze, ihre Solvate und die Solvate ihrer Salze.

Bevorzugt sind darunter besonders solche Verbindungen der Formel (I), in welchen
- Ar: für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
- R¹: für Wasserstoff, Methyl, Methoxy, Fluor oder Chlor steht,
- R²: für Wasserstoff steht,
- R³: für Methyl, iso-Propyl, tert.-Butyl, Cyano, Fluor, Chlor, Nitro oder Trifluormethyl steht,
- R⁴: für Wasserstoff steht
und
- R⁵: für Wasserstoff steht,
und ihre Salze, ihre Solvate und die Solvate ihrer Salze.

Bevorzugt sind darunter besonders auch solche Verbindungen der Formel (I), in welchen
- Ar: für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
- R¹: für Wasserstoff, Methyl, Methoxy, Fluor oder Chlor steht,
- R²: für Wasserstoff steht,
- R³: für Methyl, Cyano, Fluor, Chlor, Nitro oder Trifluormethyl steht,

- R⁴: für Wasserstoff steht
und
- R⁵: für Wasserstoff steht,
und ihre Salze, ihre Solvate und die Solvate ihrer Salze.

Bevorzugt sind auch solche Verbindungen der Formel (I), in welchen R¹ für Wasserstoff, Methyl, Methoxy oder Fluor steht.

Bevorzugt sind darunter besonders solche Verbindungen der Formel (I), in welchen R¹ für Methyl oder Methoxy steht.

Bevorzugt sind auch solche Verbindungen der Formel (I), in welchen R¹ über die ortho-Position zur Verknüpfungsstelle des Phenylrings an den Phenylring gebunden ist. Unter der Verknüpfungsstelle des mit den Resten R¹, R² und R³ substituierten Phenylrings wird im Rahmen der vorliegenden Erfindung das gemäß Formel (I) mit einem der beiden Dihydrochinazolin-Stickstoffatome verknüpfte Kohlenstoffatom des Phenylrings verstanden.

Besonders bevorzugt sind solche Verbindungen der Formel (I), in welchen R¹ für Methyl oder Methoxy steht und R¹ über die ortho-Position zur Verknüpfungsstelle des Phenylrings an den Phenylring gebunden ist.

Bevorzugt sind auch solche Verbindungen der Formel (I), in welchen R² für Wasserstoff steht.

Bevorzugt sind auch solche Verbindungen der Formel (I), in welchen R³ für Trifluormethyl, Chlor, Methyl, iso-Propyl oder tert.-Butyl steht.

Bevorzugt sind darunter besonders solche Verbindungen der Formel (I), in welchen R³ für Trifluormethyl, Chlor oder Methyl steht.

Bevorzugt sind auch solche Verbindungen der Formel (I), in welchen R¹ über die ortho-Position zur Verknüpfungsstelle des Phenylrings an den Phenylring gebunden ist und R³ über die R¹ gegenüberliegende meta-Position zur Verknüpfungsstelle des Phenylrings an den Phenylring gebunden ist.

Besonders bevorzugt sind solche Verbindungen der Formel (I), in welchen R¹ über die ortho-Position zur Verknüpfungsstelle des Phenylrings an den Phenylring gebunden ist, R³ für Trifluormethyl, Chlor oder Methyl steht und R³ über die R¹ gegenüberliegende meta-Position zur Verknüpfungsstelle des Phenylrings an den Phenylring gebunden ist.

Bevorzugt sind auch solche Verbindungen der Formel (I), in welchen R⁴ und R⁵ für Wasserstoff stehen.

Bevorzugt sind auch solche Verbindungen der Formel (I), in welchen Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), wobei Verbindungen der Formel 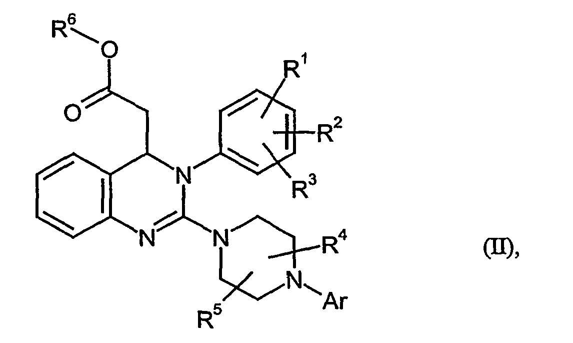 in welcher
Ar, R¹, R², R³, R⁴ und R⁵ die oben angegebene Bedeutung haben und
R⁶ für Alkyl, bevorzugt Methyl oder Ethyl, steht,
mit Basen umgesetzt werden.

Die Umsetzung erfolgt im allgemeinen in inerten Lösungsmitteln, bevorzugt in einem Temperaturbereich von Raumtemperatur bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, gegebenenfalls in wässriger Lösung, bevorzugt ist Natriumhydroxid in Wasser.

Inerte Lösungsmittel sind beispielsweise Ether wie 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Gemischen von Lösungsmitteln, bevorzugt ist Dioxan oder Tetrahydrofuran.

Die Verbindungen der Formel (II) sind bekannt oder können hergestellt werden, indem Verbindungen der Formel 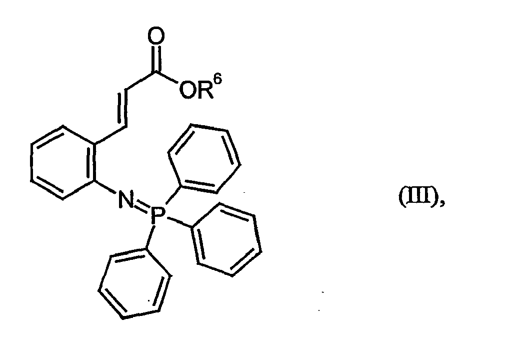 in welcher
- R⁶: die oben angegebene Bedeutung hat,
in einer zweistufigen Reaktion zunächst mit Verbindungen der Formel 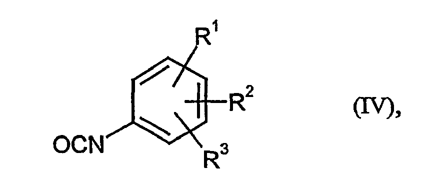 in welcher
R¹, R² und R³ die oben angegebene Bedeutung haben,
und anschließend mit Verbindungen der Formel 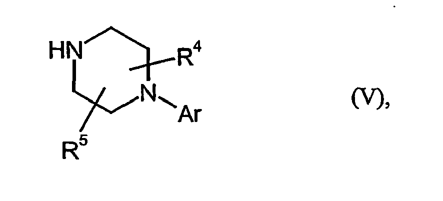 in welcher
Ar, R⁴ und R⁵ die oben angegebene Bedeutung haben,
umgesetzt werden.

Die Umsetzung erfolgt in beiden Stufen im allgemeinen in inerten Lösungsmitteln, bevorzugt in einem Temperaturbereich von Raumtemperatur bis 100°C bei Normaldruck. In der zweiten Stufe wird gegebenenfalls Kieselgel zu der Reaktionsmischung dazugegeben. Die Umsetzung erfolgt bevorzugt mit einer Aufarbeitung zwischen der ersten und der zweiten Stufe.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Essigsäureethylester, oder Gemischen von Lösungsmitteln, bevorzugt ist Methylenchlorid.

Die Verbindungen der Formel (IV) sind bekannt oder lassen sich nach bekannten Verfahren aus den entsprechenden Edukten synthetisieren.

Die Verbindungen der Formel (V) sind bekannt oder lassen sich nach bekannten Verfahren aus den entsprechenden Edukten synthetisieren, beispielsweise durch eine Buchwald-Hartwig-Reaktion nach folgendem Syntheseschema (Übersicht in: C.G. Frost, P. Mendonca, J. Chem. Soc., Perkin Trans I, 1998, 2615-2623):

### Buchwald-Hartwig-Reaktion:

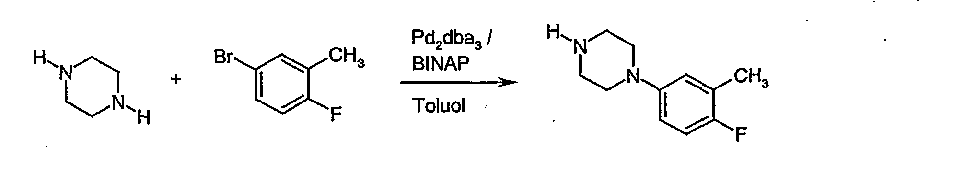

Die dafür benötigten Edukte sind bekannt oder lassen sich nach bekannten Verfahren aus den entsprechenden Edukten synthetisieren.

Die Verbindungen der Formel (III) sind bekannt oder können hergestellt werden, indem Verbindungen der Formel 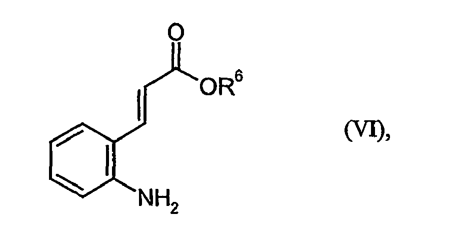 in welcher
- R⁶: die oben angegebene Bedeutung hat,
mit Triphenylphosphin und Tetrachlorkohlenstoff umgesetzt werden.

Die Umsetzung erfolgt im allgemeinen in inerten Lösungsmitteln, in Gegenwart einer Base, bevorzugt in einem Temperaturbereich von Raumtemperatur bis 50°C bei Normaldruck.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, bevorzugt ist Acetonitril.

Basen sind beispielsweise Alkali- und Erdalkalicarbonate wie Cäsiumcarbonat, Natrium- oder Kaliumcarbonat oder Amine wie Triethylamin, Diisopropylethylamin, N-Methylmorpholin oder Pyridin, bevorzugt ist Triethylamin.

Die Verbindungen der Formel (VI) sind bekannt oder lassen sich nach bekannten Verfahren aus den entsprechenden Edukten synthetisieren.

Die Herstellung der erfmdungsgemäßen Verbindungen kann durch das folgende Syntheseschema verdeutlicht werden.

### Syntheseschema:

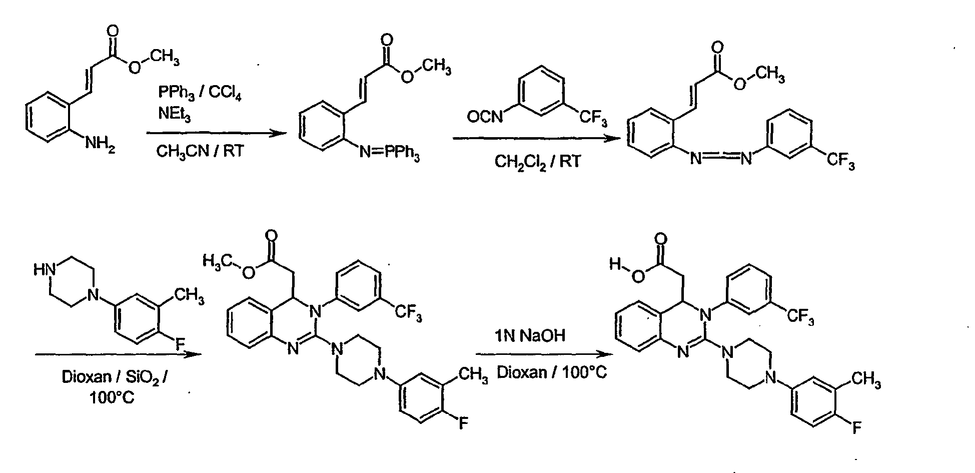

Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Injektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Injektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Gancyclovir oder Acyclovir.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfmdungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 50 mg/kg, vorzugsweise 0.1 bis 25 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- ca.: circa
- BINAP: 2,2'-Bis(diphenylphosphino)-1,1'-hinaphthyl
- CDCl₃: Deuterochloroform
- DC: Dünnschichtchromatographie
- DCI: direkte chemische Ionisation (bei MS)
- DCM: Dichlormethan
- DIEA: *N,N-*Diisopropylethylamin
- DMSO: Dimethylsulfoxid
- DMF: *N,N*-Dimethylformamid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- h: Stunde
- HPLC: Hochdruck-, Hochleistungsflüssigchromatographie
- konz.: konzentriert
- LC-MS: Flüssigchromatographie-gekoppelte Massenspektroskopie
- LDA: Lithium-Diisopropylamid
- min: Minuten
- MS: Massenspektroskopie
- NMR: Kernresonanzspektroskopie
- Pd-C: Palladium auf Kohle
- proz.: prozentig
- RP-HPLC: Reverse Phase HPLC
- RT: Raumtemperatur
- Rₜ: Retentionszeit (bei HPLC)
- THF: Tetrahydrofuran

### Allgemeine Methoden LC-MS und HPLC:

**Methode 1 (HPLC):** Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 6.5 min 90%B; Fluss: 0.75 ml/min; Temp.: 30°C; UV-Detektion: 210 nm.
**Methode 2 (HPLC, präparative Trennung):** Säule: CromSil C18, 250x30; Fluss 50 ml/min; Laufzeit: 38 min; Detektion bei 210 nm; Eluent A: Wasser, Eluent B: Acetonitril, Gradient: 10%B (3 min) -> 90%B (31 min) -> 90%B (34 min) -> 10% B (34.01 min).
**Methode 3 (HPLC, Enantiomerentrennungen):** kommerzielle CSP: Daicel Chiralpak AD mit Elutionsmittelgemischen aus i-Hexan und Alkoholen wie Ethanol und Isopropanol unter Zusatz von Diethylamin im Verhältnis 85: 15: 0.03 (v/v/v).
**Methode 4 (LCMS):** Instrument: Micromass TOF-MUX-Interface 4-fach-Parallel-Einspritzung, Waters600; Säule: YMC-ODS-AQ, 50 mm x 2.1 mm, 3.0 µm; Eluent A: Wasser + 0.05% Ameisensäure, Eluent B: Acetonitril + 0.05% Ameisensäure; Gradient: 0.0 min 100%A 〉̶ A 0.2 min 100%A 〉̶ 2.9 min 30%A 〉̶ 3.1 min 10%A 〉̶ 4.5 min 10%A; Ofen: Raumtemperatur; Fluss: 0.8 ml/min; UV-Detektion: 210 nm.
**Methode 5 (LCMS):** Instrument: Micromass Quattro LCZ, HP1100; Säule: Symmetry C18, 50 mm x 2.1 mm, 3.5 µm; Eluent A: Acetonitril + 0.1% Ameisensäure, Eluent B: Wasser + 0,1% Ameisensäure; Gradient: 0.0 min 10%A 〉̶ 4.0 min 90%A 〉̶ 6.0 min 90%A; Ofen: 40°C; Fluss: 0.5 ml/min; UV-Detektion: 208-400 nm.
**Methode 6 (LCMS):** Instrument: Finnigan MAT 900S, TSP: P4000, AS3000,UV3000HR; Säule: Symmetry C 18, 150 mm x 2.1 mm, 5.0 µm; Eluent C: Wasser, Eluent B: Wasser + 0.3g 35%ige Salzsäure, Eluent A: Acetonitril; Gradient: 0.0 min 2%A 〉̶ 2.5 min 95%A 〉̶ 5 min 95%A; Ofen: 70°C; Fluss: 1.2 ml/min; UV-Detektion: 210 nm.
**Methode 7 (HPLC):** Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 9 min 90%B; Fluss: 0.75 ml/min; Ofen: 30°C; UV-Detektion: 210 nm.

### Ausgangsverbindungen

### Allgemeine Arbeitsvorschrift [A]: Veresterung von 2-Nitrozimtsäuren mit Methanol

517.7 mmol 2-Nitrozimtsäure werden in 600 ml Methanol vorgelegt und dann mit 20 Tropfen konzentrierter Schwefelsäure versetzt und für 72 Stunden unter Rückfluss erhitzt. Nach beendeter Reaktion (Reaktionskontrolle mittels DC) wird die Reaktionslösung mit einem Eisbad gekühlt. Die entstehenden Kristalle werden abgesaugt. Die Mutterlauge wird etwas eingeengt und die dabei entstehenden Kristalle werden abgesaugt. Beide Fraktionen werden vereinigt und aus Methanol bei RT umkristallisiert.

### Beispiel 1A

### (2E)-3-(2-Nitrophenyl)-propensäuremethylester

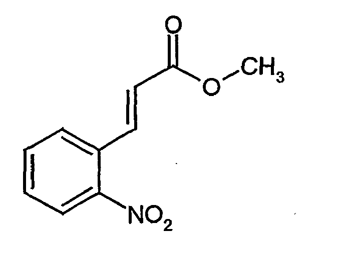

Ausgehend von 100.0 g (517.7 mmol) 2-Nitrozimtsäure werden nach der allgemeinen Arbeitsvorschrift [A] 72.6 g (68 % d. Th.) Produkt erhalten.
HPLC (Methode 1): Rₜ = 4.21 min

### Allgemeine Arbeitsvorschrift JBI: Reduktion der Nitrogrupue der 2-Nitrozimtsäurederivate

In einem 250 ml Zweihalskolben werden unter Argon in 60 ml absolutem Ethanol 25 mmol der Nitroverbindung und 125 mmol Zinn-(II)-chloriddihydrat vorgelegt. Diese Suspension wird 30 Minuten unter Rückfluss gerührt, und es entsteht eine klare Lösung. Dann lässt man die Lösung auf Raumtemperatur abkühlen und gießt sie danach auf Eiswasser. Der pH-Wert wird entweder mit festem Natriumhydrogencarbonat oder mit einer gesättigten Natriumcarbonat-Lösung auf pH 7-8 eingestellt. Anschließend gibt man 60 ml Ethylacetat hinzu und filtriert die ausgefallenen Zinnsalze über ca. 1 cm Schichtdicke Kieselgur ab. Die organische Phase wird abgetrennt und die wässrige Phase noch einmal mit Ethylacetat extrahiert. Man vereinigt die organischen Phasen und wäscht sie einmal mit gesättigter Natriumchlorid-Lösung, trocknet sie über Natriumsulfat und engt das Lösemittel ca. um die Hälfte ein. Nun fügt man Aktivkohle hinzu, entsprechend 1 % des Gewichts der Nitroverbindung, und erhitzt für 30 Minuten unter Rückfluss (Verfärbung der Lösung). Die Aktivkohle wird abfiltriert und das Lösemittel eingeengt. Der Rückstand wird im Hochvakuum getrocknet und ohne weitere Aufreinigung erfolgt eine direkte Umsetzung zur nächsten Stufe.

### Beispiel 2A

### (2E)-3-(2-Aminophenyl)-propensäuremethylester

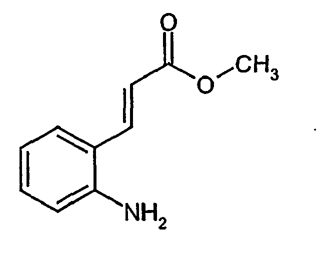

Ausgehend von 15.00 g (72.34 mmol) Nitroverbindung werden nach der allgemeinen Arbeitsvorschrift [B] 12.05 g (94 % d. Th.) Produkt erhalten.
HPLC (Methode 5): Rₜ = 3.29 min

### Allgemeine Arbeitsvorschrift [C]: Synthese der Iminophosphorane mittels Appel-Reaktion der substituierten Aniline

In einem 50 ml Einhalskolben werden 10.0 mmol des 2-Aminozimtsäureesters, 20.0 mmol Triphenylphosphin, 100.0 mmol Tetrachlorkohlenstoff und 100.0 mmol Triethylamin in 20 ml Acetonitril gelöst. Man lässt 2 Stunden bei Raumtemperatur rühren. Nach beendeter Reaktion (Reaktionskontrolle mittels DC oder analytischer HPLC) wird das Lösungsmittel im Vakuum entfernt und der Rückstand durch Säulenchromatographie an Kieselgel mit Cyclohexan/Ethylacetat = 7:3 gereinigt.

### Beispiel 3A

### (2E)-3-{2-[(Triphenylphosphoranyliden)amino]phenyl}-propensäuremethylester

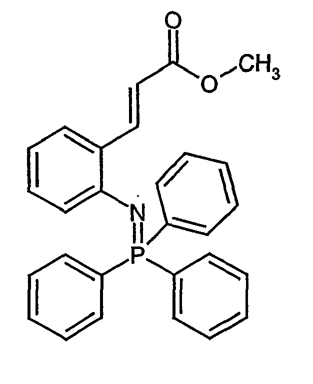

Ausgehend von 2.00 g (11.28 mmol) Aminverbindung werden mit 5.92 g (22.57 mmol) Triphenylphosphin nach der allgemeinen Arbeitsvorschrift [C] 4.42 g (90 % d. Th.) Produkt erhalten.
HPLC (Methode 6): Rₜ = 2.00 min
MS (ESIpos): m/z = 428 (M+H)⁺

### Allgemeine Arbeitsvorschrift [D]: Synthese von Phenylpiperazinen via Buchwald-Hartwig-Reaktion

Zur Reaktionsvorbereitung wird der Reaktionskolben am Hochvakuum gründlich ausgeheizt und beim Belüften mit Argon gefüllt. In den Kolben werden 1.0 Äquivalente Bromarlyverbindung und 6.0 Äquivalente Piperazin in absolutem Toluol vorgelegt (0.2-0.3M Lösung der Bromverbindung). Dann werden 0.01 Äquivalente Tris(dibenzylidenaceton)dipalladium sowie 0.03 Äquivalente BINAP zugegeben. Das Reaktionsgemisch wird 16 h unter Rückfluss gerührt. Anschließend wird der Ansatz einmal mit Wasser extrahiert, die organische Phase zweimal mit 1N Salzsäure extrahiert, die wässrige Phase mit 1N Natronlauge auf pH 8 gestellt und dreimal mit Dichlormethan extrahiert. Die vereinigten organischen Phasen werden über Natriumsulfat getrocknet, filtriert, das Lösungsmittel im Vakuum entfernt und das Produkt über Nacht im Hochvakuum getrocknet.

### Beispiel 4A

### N-(4-Fluor-3-methylphenyl)-piperazin

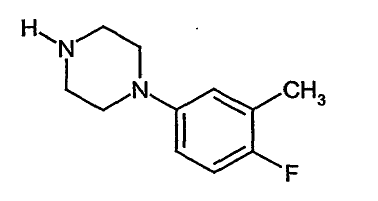

Ausgehend von 5.0 g (26.5 mmol) 4-Fluor-3-methyl-1-brombenzol werden nach der allgemeinen Arbeitsvorschrift [D] 4.52 g (83% d. Th.) Produkt erhalten.
HPLC (Methode 1): Rₜ = 3.54 min
MS (ESI pos): m/z = 195 (M+H)⁺

### Allgemeine Arbeitsvorschrift [E]: Umsetzung des Iminophosphorans mit einem Isocyanat und anschließende Umsetzung zum Dihydrochinazolin-Derivat mit einem Amin

1.0 Äquivalente des Iminophosphorans werden in 20 ml Dichlormethan gelöst (0.1-0.2M Lösung). Danach werden 1.05 Äquivalente eines substituierten Isocyanats hinzugefügt und man lässt bis zur Beendigung der Reaktion bei RT rühren. Eine Reaktionskontrolle erfolgt durch DC oder analytische HPLC.

Die so erhaltene Lösung des Carbodiimids in Dichlormethan wird mit 1.0 Äquivalenten Amin sowie einer Spatelspitze Kieselgel versetzt und bei Raumtemperatur bis zur vollständigen Umsetzung gerührt. Nach beendeter Reaktion (Reaktionskontrolle mittels DC oder HPLC) wird der Ansatz eingeengt und durch präparative HPLC an RP-Phase gereinigt.

Unter Umständen zeigt das NMR noch einen schwankenden Anteil an nicht-cyclisiertem Reaktionsprodukt an. In diesen Fällen wird das Gemisch aus cyclisiertem und nicht-cyclisiertem Produkt in Dioxan aufgenommen, mit einer Spatelspitze Kieselgel versetzt und unter Rückfluss 30 min bis 16 h gerührt. Das Kieselgel wird abfiltriert und die Lösung für weitere Umsetzungen verwendet.

### Beispiel 5A

### {2-[4-(4-Fluorphenyl)-1-piperazinyl]-3-[3-(trifluormethyl)phenyl]-3,4-dihydro-4-chinazolinyl}essigsäuremethylester

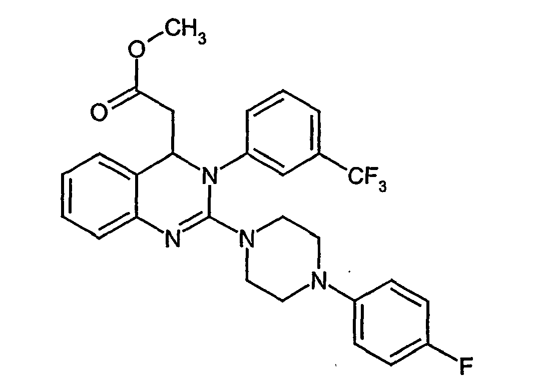

Ausgehend von 5.0 g (11.43 mmol) Iminophosphoran aus Beispiel 3A, 2.25 g (12.0 mmol) Trifluor-m-toloylisocyanat und 2.06 g (11.43 mmol) N-(4-Fluorphenyl)-piperazin werden nach der allgemeinen Arbeitsvorschrift [E] 3.31 g (39 % d. Th.) Produkt erhalten.
HPLC (Methode 1): Rₜ = 4.72 min
MS (ESI pos): m/z = 527 (M+H)⁺

### Beispiel 6A

### {2-[4-(4-Fluorphenyl)-1-piperazinyl]-3-[3-(trifluormethyl)phenyl]-3,4-dihydro-4-chinazolinyl}essigsäuremefhylester

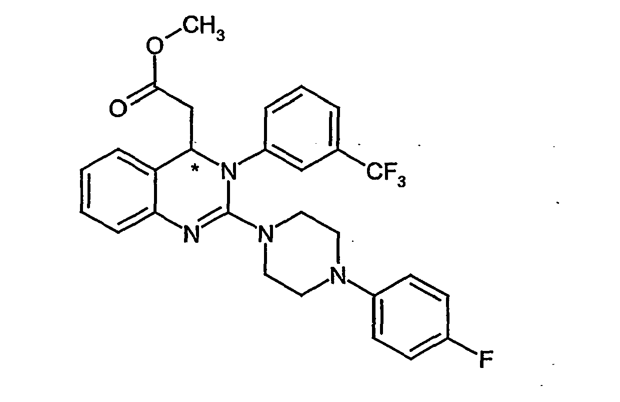

Ausgehend von 300 mg des Methylesters aus Beispiel 5A werden nach Enatiomerentrennung (gemäß Methode 3) 125 mg des Enantiomers A erhalten.
α_{D}²⁰=+196.6 (C = 0.53, CHCl₃)

### Beispiel 7A

### {2-[4-(4-Fluor-3-methylphenyl)-1-piperazinyl]-3-[3-(trifluormethyl)phenyl]-3,4-dihydro-4-chinazolinyl} essigsäuremethylester

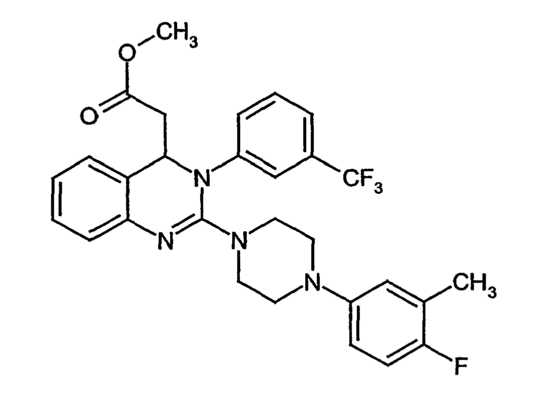

Ausgehend von 200 mg (0.46 mmol) Iminophosphoran aus Beispiel 3A, 90 mg (0.48 mmol) Trifluor-m-toloylisocyanat und 89 mg (0.46 mmol) des Phenylpiperazins aus Beispiel 4A werden nach der allgemeinen Arbeitsvorschrift [E] und nach chromatographischer Reinigung (Methode 2) 112 mg (43 % d. Th.) Produkt erhalten.
HPLC (Methode 1): Rₜ = 4.96 min
MS (ESI pos): m/z = 541 (M+H)⁺

### Beispiel 8A

### {2-[4-(4-Fluorphenyl)piperazin-1-yl]-3-[2-fluor-5-(trifluorinethyl)phenyl]-3,4-dihydro-chinazolin-4-yl} essigsäuremethylester

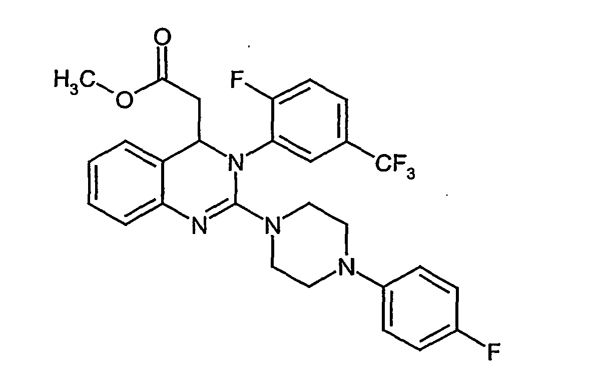

Ausgehend von 150 mg (0.34 mmol) Iminophosphoran aus Beispiel 3A, 74 mg (0.34 mmol) 2-Fluor-5-(trifluormethyl)phenylisocyanat und 61 mg (0.34 mmol) N-(4-Fluorphenyl)-piperazin werden nach der allgemeinen Arbeitsvorschrift [E] und nach chromatographischer Reinigung (Methode 2) 34 mg (17% d. Th.) Produkt erhalten.
HPLC (Methode 1): Rₜ = 4.73 min
MS (ESI pos): m/z = 545 (M+H)⁺

### Beispiel 9A

### {2-[4-(3-Chlorphenyl)piperazin-1-yl]-3-[2-fluor-5-(trifluormethyl)phenyl]-3,4-dihydro-chinazolin-4-yl} essigsäuremethylester

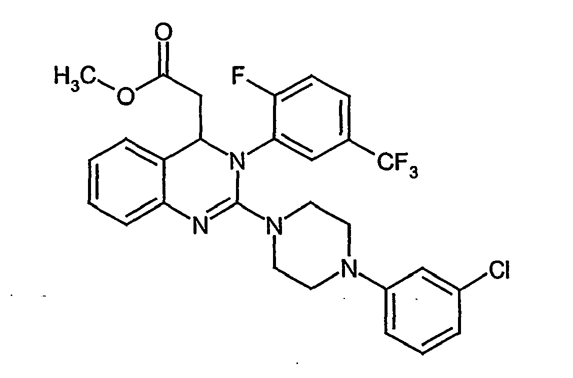

Ausgehend von 150 mg (0.34 mol) Iminophosphoran aus Beispiel 3A, 74 mg (0.34 mmol) 2-Fluor-5-(trifluormethyl)phenylisocyanat und 67 mg (0.34 mmol) N-(3-Chlorphenyl)-piperazin werden nach der allgemeinen Arbeitsvorschrift [E] und nach chromatographischer Reinigung (Methode 2) 95 mg (50% d. Th.) Produkt erhalten.
HPLC (Methode 1): Rₜ = 4.91 min
MS (ESI pos): m/z = 561 (M+H)⁺

### Beispiel 10A

### {2-[4-(4-Fluorphenyl)piperazin-1-yl]-3-[4-fluor-5-(trifluormethyl)phenyl]-3,4-dihydro-chinazolin-4-yl} essigsäuremethylester

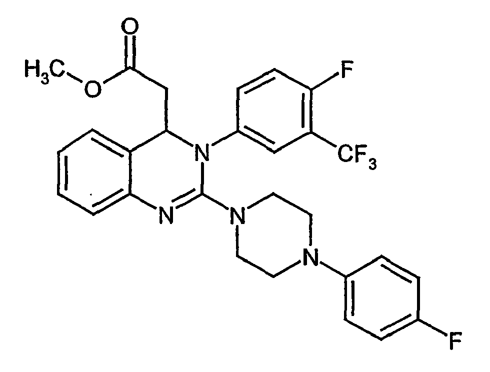

Ausgehend von 150 mg (0.34 mmol) Iminophosphoran aus Beispiel 3A, 74 mg (0.34 mmol) 4-Fluor-3-(trifluormethyl)phenylisocyanat und 61 mg (0.34 mmol) N-(4-Fluorphenyl)-piperazin werden nach der allgemeinen Arbeitsvorschrift [E] und nach chromatographischer Reinigung (Methode 2) 111 mg (54% d. Th.) Produkt erhalten.
HPLC (Methode 1): Rₜ = 4.87 min
MS (ESI pos): m/z = 545 (M+H)⁺

### Beispiel 11A

### {2-[4-(3-Chlorphenyl)piperazin-1-yl]-3-[2-methyl-4-chlorphenyl]-3,4-dihydro-chinazolin-4-yl}essigsäuremethylester

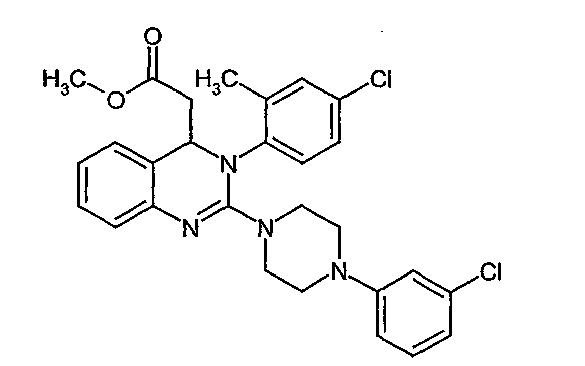

Ausgehend von 150 mg (0.34 mmol) Iminophosphoran aus Beispiel 3A, 60 mg (0.36 mmol) 4-Chlor-2-methyl-phenylisocyanat und 67 mg (0.34 mmol) N-(3-Chlorphenyl)-piperazin werden nach der allgemeinen Arbeitsvorschrift [E] und nach chromatographischer Reinigung (Methode 2) 97 mg (46% d. Th.) Produkt erhalten.
HPLC (Methode 1): Rₜ = 5.03 min
MS (ESI pos): m/z = 523 (M+H)⁺

### Beispiel 12A

### 2-Isocyanato-1-methoxy-4-(trifluormethyl)benzol

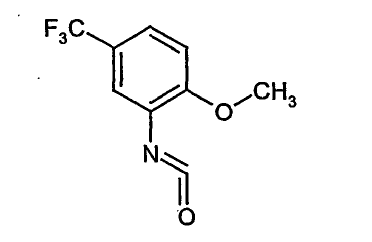

In 100 ml Dichlormethan werden 3 g (15.69 mmol) 2-Methoxy-5-trifluormethylanilin gelöst und mit 6.73 g (31.39 mmol) 1,8-Bis(dimethylamino)naphthalin versetzt. Bei 0-5°C werden 2.24 g (11.3 mmol) Chlorameisensäuretrichlormethylester, gelöst in 50 ml Dichlormethan, zugetropft und 30 min bei 0°C sowie 60 min bei Raumtemperatur gerührt. Bei 0°C wird mit 1N Salzsäure, Eiswasser und Natriumhydrogencarbonat-Lösung gewaschen. Nach Trocknen über Magnesiumsulfat und Abdestillieren des Lösungsmittels wird das Produkt erhalten. Das Isocyanat wird anschließend ohne weitere Reinigung in den folgenden Reaktionen umgesetzt.
Ausbeute: 3 g (88% d. Th.)

### Allgemeine Arbeitsvorschrift [G]: Umsetzung des Iminophosphorans mit einem Isocyanat zu einem Carbodiimid

1.0 Äquivalente des Iminophosphorans werden in 20 ml Dichlormethan gelöst (0.1-0.2M Lösung). Danach werden 1.05 Äquivalente eines Isocyanats hinzugefügt und man lässt bis zur Beendigung der Reaktion bei RT rühren. Eine Reaktionskontrolle erfolgt durch DC oder analytische HPLC. Anschließend wird das Lösungsmittel im Vakuum entfernt und das Rohprodukt mittels Chromatographie an Kieselgel mit Cyclohexan/Dichlormethan-Gemischen gereinigt.

### Beispiel 13A

### (2E)-3-{2-[(Iminomethylen)amino]phenyl}acrylsäuremethylester-1-methoxy-2-methyl-4-(trifluoromethyl)benzol

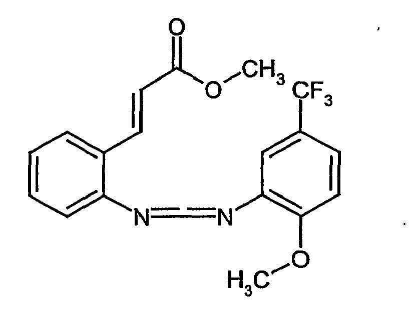

Ausgehend von 2.0 g (4.57 mmol) Iminophosphoran aus Beispiel 3A und 1.04 g (4.8 mmol) Isocyanat aus Beispiel 12A werden nach der allgemeinen Arbeitsvorschrift [G] und nach Chromatographie mit Cyclohexan/Dichlormethan (2:1 v/v, dann 1/1 v/v) 0.79 g (38% d. Th.) Produkt erhalten.
HPLC (Methode 1): Rₜ = 5.52 min

### Beispiel 14A

### (2E)-3-{2-[(Iminomethylen)amino]phenyl}acrylsäuremethylester-1-methoxy-2-methyl-4-chlorbenzol

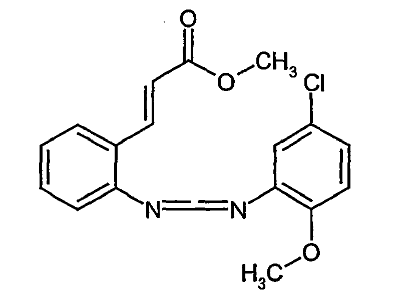

Ausgehend von 2.0 g (4.57 mmol) Iminophosphoran aus Beispiel 3A und 0.88 g (4.8 mmol) 2-Methoxy-5-chlorphenylisocyanat werden nach der allgemeinen Arbeitsvorschrift [G] und nach Chromatographie mit Cyclohexan/Dichlormethan (2:1 v/v, dann 1:1 v/v) 0.67 g (34% d. Th.) Produkt erhalten.
HPLC (Methode 1): Rₜ = 5.53 min

### Beispiel 15A

### (2E)-3-{2-[(Iminomethylen)amino]phenyl}acrylsäuremethylester-1-methoxy-2-methyl-4-methylbenzol

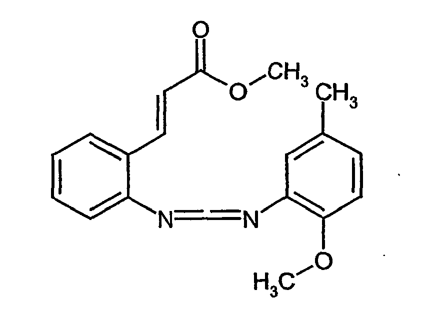

Ausgehend von 2.0 g (4.57 mmol) Iminophosphoran aus Beispiel 3A und 0.78 g (4.8 mmol) 2-Methoxy-5-methylphenylisocyanat werden nach der allgemeinen Arbeitsvorschrift [G] und nach Chromatographie mit Cyclohexan/Dichlormethan (2:1 v/v, dann 1/1 v/v) 0.85 g (57% d. Th.) Produkt erhalten.
HPLC (Methode 1): Rₜ = 5.45 min

### Allgemeine Arbeitsvorschrift [H]: Umsetzung_eines Carbodiimids mit einem Phenylpiperazin zum Chinazolin

1.0 Äquivalente des Carbodiimids werden in Dioxan gelöst (0.1-0.25M Lösung). Danach werden 1.0 Äquivalente des Phenylpiperazins hinzugefügt, der Ansatz mit Kieselgel versetzt und das Gemisch unter Rückfluss des Lösungsmittels gerührt. Eine Reaktionskontrolle erfolgt durch DC oder analytische HPLC. Anschließend wird das Lösungsmittel im Vakuum entfernt und das Rohprodukt mittels Chromatographie an Kieselgel mit Cyclohexan/Ethylacetat-Gemischen oder mittels präparativer HPLC (Methode 2) gereinigt.

### Beispiel 16A

### {2-[4-(3-Chlorphenyl)-1-piperazinyl]-3-[2-methoxy-5-(trifluormethyl)phenyl]-3,4-dihydro-4-chinazolinyl} -essigsäuremethylester

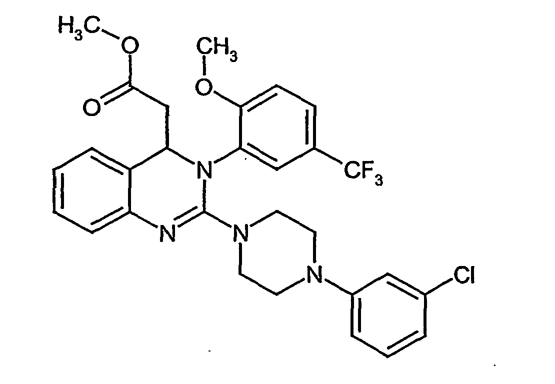

Ausgehend von 160 mg (0.43 mmol) Carbodiimid aus Beispiel 13A und 83.6 mg (0.43 mmol) 3-Chlorphenylpiperazin werden nach der allgemeinen Arbeitsvorschrift [H] 148 mg (61% d. Th.) Produkt erhalten.
HPLC (Methode 1): Rₜ = 4.88 min

### Beispiel 17A

### {2-[4-(3-Methylphenyl)-1-piperazinyl]-3-[2-methoxy-5-(trifluormethyl)phenyl]-3,4-dihydro-4-chinazolinyl}-essigsäuremethylester

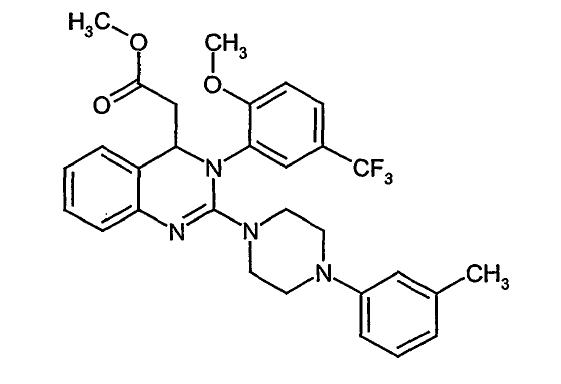

Ausgehend von 150 mg (0.40 mmol) Carbodiimid aus Beispiel 13A und 70 mg (0.40 mmol) 3-Methylphenylpiperazin werden nach der allgemeinen Arbeitsvorschrift [H] 159 mg (72% d. Th.) Produkt erhalten.
HPLC (Methode 1): Rₜ = 4.79 min

### Beispiel 18A

### {2-[4-(3-Methoxyphenyl)-1-piperazinyl]-3-[2-methoxy-5-chlorphenyl]-3,4-dihydro-4-chinazolinyl}-essigsäuremethylester

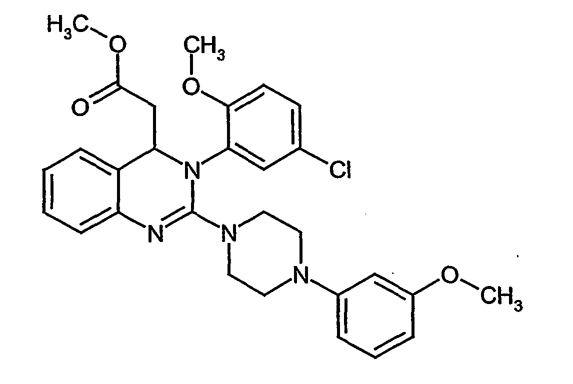

Ausgehend von 100 mg (0.29 mmol) Carbodiimid aus Beispiel 14A und 56 mg (0.29 mmol) 3-Methoxyphenylpiperazin werden nach der allgemeinen Arbeitsvorschrift [H] 115 mg (74% d. Th.) Produkt erhalten.
HPLC (Methode 1): Rₜ = 4.7 min

### Beispiel 19A

### {2-[4-(4-Fluorphenyl)-1-piperazinyl]-3-[2-methoxy-5-chlorphenyl]-3,4-dihydro-4-chinazolinyl}-essigsäuremethylester

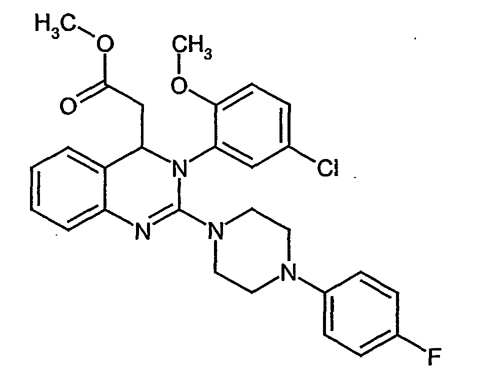

Ausgehend von 100 mg (0.29 mmol) Carbodiimid aus Beispiel 14A und 53 mg (0.29 mmol) 4-Fluorphenylpiperazin werden nach der allgemeinen Arbeitsvorschrift [H] 108 mg (71% d. Th.) Produkt erhalten.
HPLC (Methode 1): Rₜ = 4.68 min

### Beispiel 20A

### {2-[4-(3-Methylphenyl)-1-piperazinyl]-3-[2-methoxy-5-methylphenyl]-3,4-dihydro-4-chinazolinyl}-essigsäuremethylester

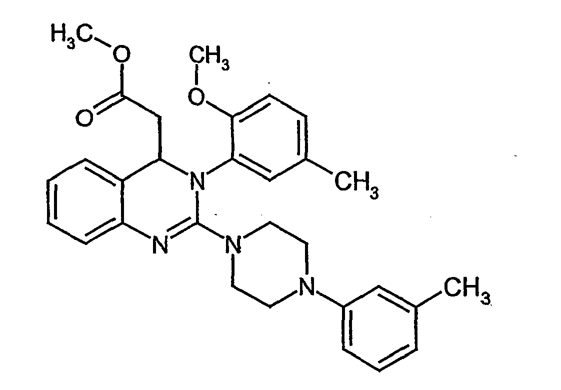

Ausgehend von 160 mg (0.50 mmol) Carbodiimid aus Beispiel 15A und 87 mg (0.50 mmol) 3-Methylphenylpiperazin werden nach der allgemeinen Arbeitsvorschrift [H] 205 mg (83 d. Th.) Produkt erhalten.
HPLC (Methode 1): Rₜ = 4.93 min

### Beispiel 21A

### {2-[4-(3-Chlorphenyl)-1-piperazinyl]-3-[2-methoxy-5-methylphenyl]-3,4-dihydro-4-chinazolinyl} -essigsäuremethylester

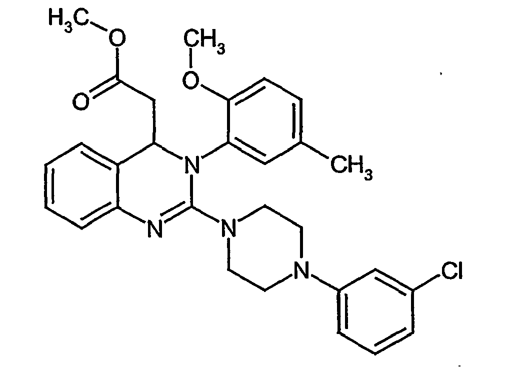

Ausgehend von 160 mg (0.50 mmol) Carbodiimid aus Beispiel 15A (WTB3297) und 98 mg (0.50 mmol) 3-Chlorphenylpiperazin werden nach der allgemeinen Arbeitsvorschrift [H] 207 mg (80% d. Th.) Produkt erhalten.
HPLC (Methode 1): Rₜ = 5.04 min

### Ausführungsbeispiele

### Allgemeine Arbeitsvorschrift [F]: Esterverseifung der Chinazolylessigsäurester

Es werden 1.0 Äquivalente des Chinazolylessigsäuresters in Dioxan gelöst und 5.0 Äquivalente 1N Natronlauge hinzugefügt. Man lässt für 16 Stunden bei 100°C rühren und nach beendeter Reaktion (Reaktionskontrolle mittels analytischer HPLC) wird der Ansatz eingeengt. Der Rückstand wird dann in Wasser aufgenommen und mit 1N Salzsäure auf pH 5 gestellt. Man filtriert den entstehenden Niederschlag ab, wäscht ihn mit wenig Wasser und Diethylether und trocknet ihn im Hochvakuum bei Raumtemperatur. Falls die Reinheit des Produktes nicht hoch genug ist, wird es über präparative HPLC an RP-Phase gereinigt (Methode 2).

### Beispiel 1

### {2-[4-(4-Fluor-3-methylphenyl)-1-piperazinyl]-3-[3-(trifluormethyl)phenyl]-3,4-dihydro-4-chinazolinyl}essigsäure

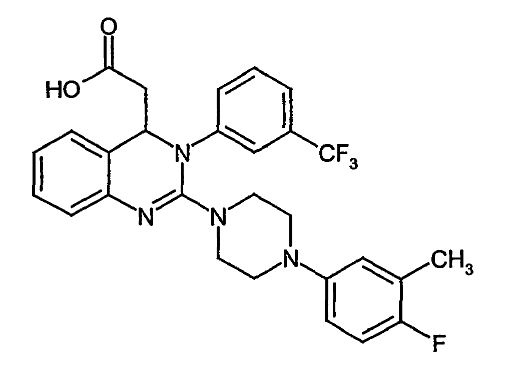

Ausgehend von 98 mg (0.181mmol) Methylester aus Beispiel 7A werden nach der allgemeinen Arbeitsvorschrift [F] 66.4 mg (61% d. Th.) Produkt erhalten.
HPLC (Methode 1): Rₜ = 4.69 min
MS (ESIpos): m/z = 527 (M+H)⁺
¹H-NMR (400 MHz, CD₃CN): δ [ppm] = 7.75 (s, 1H), 7.67, (d, 1H), 7.57-7.56 (m, 3H), 7.37 (dt, 1H), 7.22-7.15 (m, 2H), 6.87 (t, 1H), 6.73 (dd, 1H), 6.64-6.60 (m, 1H), 5.36-5.32 (m, 1H), 3.70-3.53 (m, 4H), 3.10-2.96 (m, 5H), 2.67 (dd, 1H), 2.18 (d, 3H).

### Beispiel 2

### {2-[4-(4-Fluorphenyl)-1-piperazmyl]-3-[2-fluor-5-(trifluormethyl)phenyl]-3,4-dihydro-4-chinazolinyl}essigsäure-Hydrochlorid

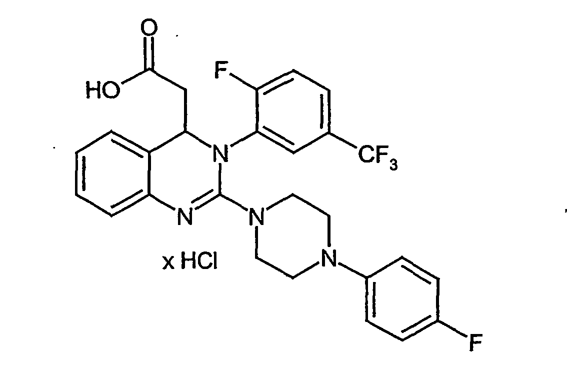

Ausgehend von 30 mg (0.055 mmol) des entsprechenden Methylesters werden nach der allgemeinen Arbeitsvorschrift [F] 20 mg (56 % d.Th.) Produkt erhalten.
HPLC (Methode 1): Rₜ = 4.5 min
MS (ESIpos): m/z = 531 (M+H)⁺
¹H-NMR (400 MHz, CD₃CN): δ [ppm] = 8.11 (d, 1H); 7.59-7.56 (m, 1H); 7.31-7.23 (m, 3H); 7.12 (d, 1H); 7.04 (t, 1H); 6.96 (t, 2H); 6.83-6.79 (m, 2H); 5.12 (t, 1H); 3.59-3.48 (m, 4H); 2.92-2.80 (m, 5H); 2.59 (dd, 1H).

### Beispiel 3

### {2-[4-(3-Chlorphenyl)-1-piperazinyl]-3-[2-fluor-5-(trifluormethyl)phenyl]-3,4-dihydro-4-chinazolinyl}essigsäure-Hydrochlorid

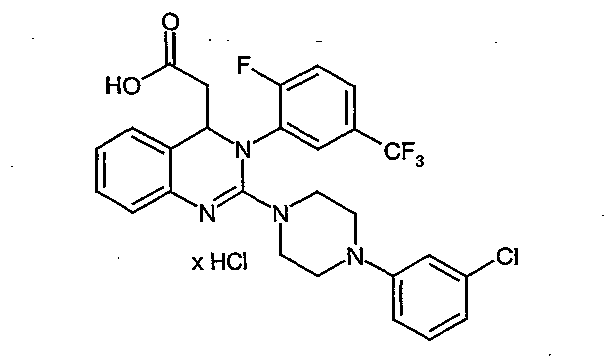

Ausgehend von 90 mg (0.16 mmol) des entsprechenden Methylesters werden nach der allgemeinen Arbeitsvorschrift [F] 24 mg (26 % d. Th.) Produkt erhalten.
HPLC (Methode 1): Rₜ = 4.63 min
MS (ESIpos): m/z = 547 (M+H)⁺
¹H-NMR (400 MHz, CD₃CN): δ [ppm] = 7.87 (d, 1H); 7.50-7.48 (m, 1H); 7.26-7.21 (m, 2H); 7.17 (t, 1H); 7.14-7.12 (m, 1H); 7.05 (dd, 1H); 6.97 (dt, 1H); 6.84 (t, 1H); 6.80-6.77 (m, 2H); 5.01 (dd,1H); 3.57-3.42 (m, 4H); 3.05-2.99,2.97-2.85 (2x m, 4H); 2.79 (dd, 1H); 2.53 (dd, 1H).

### Beispiel 4

### {2-[4-(4-Fluorphenyl)-1-piperazinyl]-3-[4-fluor-3-(trifluormethyl)phenyl]-3,4-dihydro-4-chinazolinyl} essigsäure-Hydrochlorid

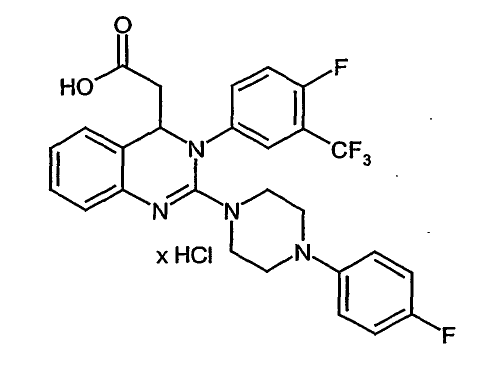

Ausgehend von 95 mg (0.17 mmol) des entsprechenden Methylesters werden nach der allgemeinen Arbeitsvorschrift [F] 27 mg (26 % d. Th.) Produkt erhalten.
HPLC (Methode 1): Rₜ = 4.56 min
MS (ESIpos): m/z = 531 (M+H)⁺
¹H-NMR (400 MHz, CD₃CN)- δ [ppm] = 7.64-7.62 (m, 1H); 7.58 (s, 1H); 7.36-7.31 (m, 1H); 7.24-7.16 (m, 2H); 7.11 (d, 1H); 7.05 (d, 1H); 7.01-6.95 (m, 3H); 6.91-6.87 (m, 2H); 5.12 (dd, 1H); 3.59-3.49 (m, 4H); 3.01-2.85 (m, 4H); 2.70 (dd, 1H); 2.53 (dd, 1H).

### Beispiel 5

### {2-[4-(4-Fluorphenyl)-1-piperazinyl]-3-[3-(trifluormethyl)phenyl]-3,4-dihydro-4-chinazolinyl} essigsäure-Hydrochlorid

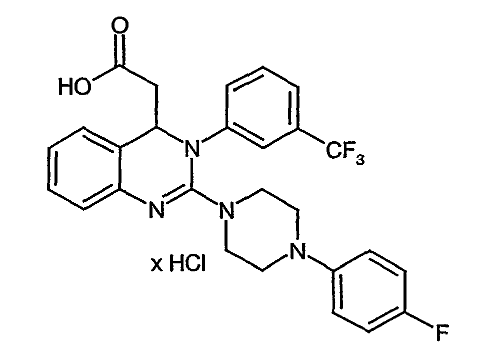

Ausgehend von 3.31 g (6.29 mmol) des Methylesters aus Beispiel 5A werden nach der allgemeinen Arbeitsvorschrift [F] 2.68 g (83 % d. Th.) Produkt erhalten.
HPLC (Methode 1): Rₜ = 4.62 min
MS (ESIpos): m/z = 513 (M+H)⁺
¹H-NMR (400 MHz, CD₃CN): δ [ppm] = 7.57 (s, 1H); 7.45 (t, 1H); 7.37-7.34 (t, 2H); 7.21 (t, 1H); 7.10 (d, 1H); 7.06 (d, 1H); 7.00-6.92 (m, 3H); 6.89-6.86 (m, 2H); 5.19 (m, 1H); 3.60-3.49 (m, 4H); 3.01-2.87 (m, 4H); 2.72 (dd, 1H); 2.54 (dd, 1H).

### Beispiel 6

### {2-[4-(3-Chlorphenyl)-1-piperazinyl]-3-[4-chlor-2-methylphenyl]-3,4-dihydro-4-chinazolinyl}essigsäure

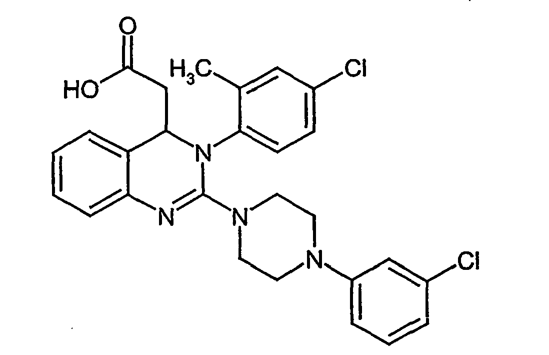

Ausgehend von 90 mg (0.172 mmol) des entsprechenden Methylesters werden nach der allgemeinen Arbeitsvorschrift [F] 71 mg (70 % d. Th.) Produkt erhalten.
HPLC (Methode 1): Rₜ = 4.75 min
MS (ESIpos): m/z = 509 (M+H)⁺
¹H-NMR (400 MHz, CD₃CN): δ [ppm] = 7.72-7.63 (m, 2H); 7.41-7.34 (m, 2H); 7.21-7.14 (m, 6H); 6.80-6.72 (m, 4H); 5.10-5.06 (m, 1H); 3.59 (s, 4H); 3.13-2.91 (m, 5H); 2.74-2.68 (m, 1H); 1.68 (s, 3H).

### Beispiel 7.

### {2-[4-(4-Fluorphenyl)-1-piperazinyl]-3-[3-(trifluormethyl)phenyl]-3,4-dihydro-4-chinazolinyl} essigsäure-Hydrochlorid

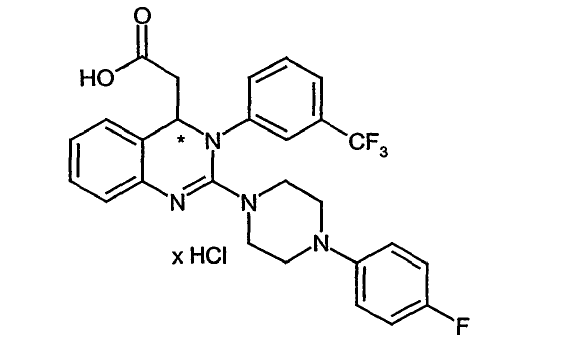

Ausgehend vorn 120 mg (0.23 mmol) des Methylesters aus Beispiel 6A werden nach der allgemeinen Arbeitsvorschrift [F] 100 mg Produkt (81 % d. Th.) erhalten.
HPLC (Methode 1): Rₜ = 4.62 min
MS (ESIpos): m/z = 513 (M+H)⁺

Die Beispiele 8 bis 25 der Tabelle 1 können nach der allgemeinen Arbeitsvorschrift [F] hergestellt werden.

**Tabelle 1**

| **Bsp-Nr.** | **Struktur** | **MW** | **Rₜ[min]** | **HPLC Methode** | **MS** |
|---|---|---|---|---|---|
| 8 | 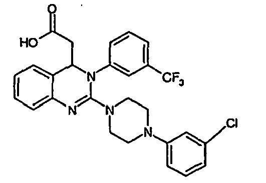 | 529.0 | 3.22 | 4 | 529 (M+H) |
| 9 | 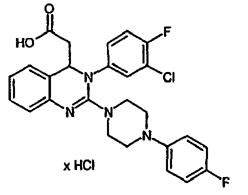 | 533.4 | 4.63 | 1 | 497 (M-HCl+H) |
| 10 | 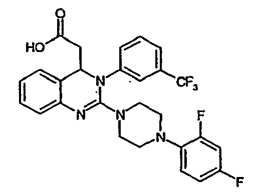 | 530.5 | 3.21 | 4 | 531 (M+H) |
| 11 | 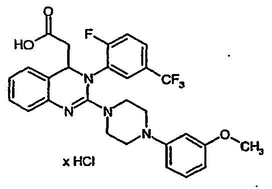 | 579.0 | 3.40 | 5 | 542 (M-HCl+H) |
| 12 | 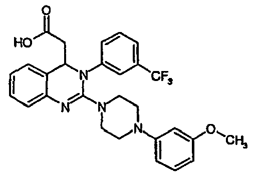 | 524.5 | 3.13 | 4 | 525 (M+H) |
| 13 | 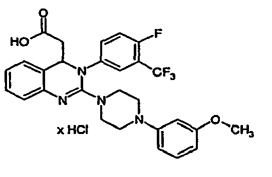 | 579.0 | 4.54 | 1 | 543 (M-HCl+H) |
| 14 | 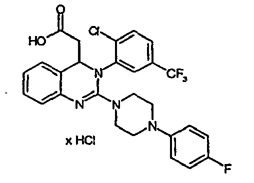 | 583.4 | 4.58 | 1 | 547 (M-HCl+H) |
| 15 | 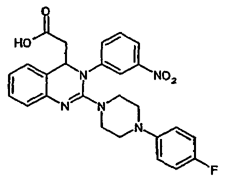 | 489.5 | 4.26 | 1 | 490 (M+H) |
| 16 | 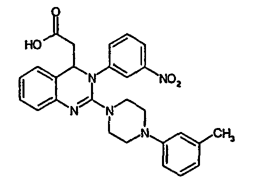 | 508.5 | 3.23 | 4 | 509 (M+H) |
| 17 | 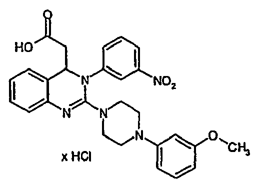 | 538.0 | 4.27 | 1 | 502 (M-HCl+H) |
| 18 | 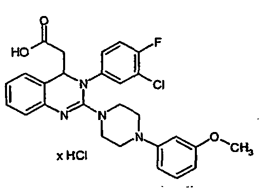 | 545.4 | 4.61 | 1 | 509 (M-HCl+H) |
| 19 | 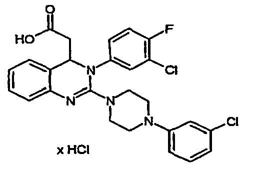 | 549.9 | 4.81 | 1 | 513 (M-HCl+H) |
| 20 | 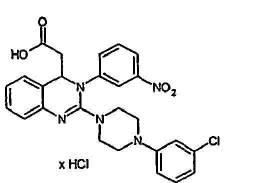 | 542.4 | 4.46 | 1 | 506 (M-HCl+H) |
| 21 | 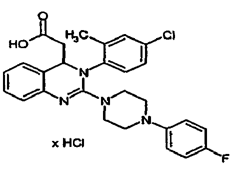 | 529.4 | 4.57 | 1 | 493 (M-HCl+H) |
| 22 | 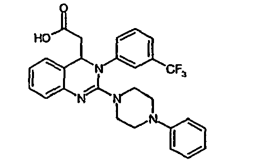 | 494.5 | 3.12 | 4 | 495 (M+H) |
| 23 | 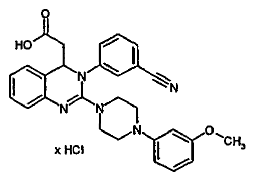 | 518.0 | 4.19 | 1 | 482 (M-HCl+H) |
| 24 | 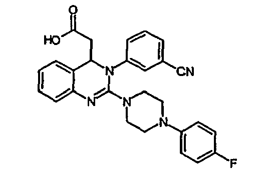 | 469.5 | 4.18 | 1 | 470 (M+H) |
| 25 | 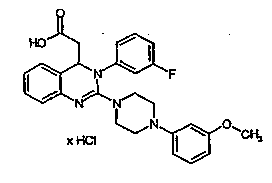 | 511.0 | 4.46 | 1 | 475 (M-HCl+H) |

### Beispiel 26

### {2-[4-(3-Chlorphenyl)-1-piperazinyl]-3-[2-methoxy-5(-trifluormethyl)phenyl]-3,4-dihydro-4-chinazolinyl}essigsäure

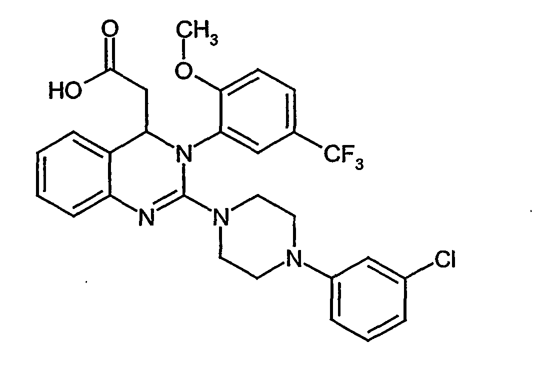

Ausgehend von 135 mg (0.24 mmol) Methylester aus Beispiel 16A werden nach der allgemeinen Arbeitsvorschrift [F] und Reinigung mittels präparativer HPLC 106 mg (80% d. Th.) Produkt erhalten.
HPLC (Methode 1): Rₜ = 4.82 min
MS (ESI-pos): m/z = 559 (M+H)⁺
¹H-NMR (400MHz, CD₃CN): δ [ppm] = 8.21 (s, 0.5H); 7.77 (s_{b}, 0.5H); 7.52 (d, 1H); 7.22-7.07 (m, 5H); 6.98 (t, 1H); 6.79-6.76 (m, 2H); 6.71 (d, 1H); 4.96 (t, 1H); 3.76 (s_{b}, 3H); 3.49-3.33 (m, 6H); 2.98-2.92 (m, 2H); 2.84-2.78 (m, 2H).

### Beispiel 27

### {2-[4-(3-Methylphenyl)-1-piperazmyl]-3-[2-methoxy-5-(trifluormethyl)phenyl]-3,4-dihydro-4-chinazolinyl}essigsäure

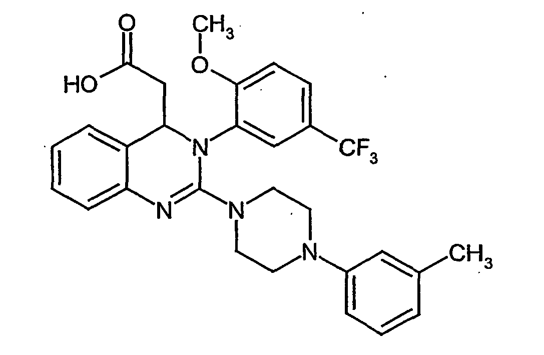

Ausgehend von 120 mg (0.2 mmol) Methylester aus Beispiel 17A werden nach der allgemeinen Arbeitsvorschrift [F] und Reinigung mittels präparativer HPLC 55 mg (47% d. Th.) Produkt erhalten.
HPLC (Methode 7): Rₜ = 4.57 min
MS (ESI-neg): m/z = 537 (M-H)⁻
¹H-NMR (400MHz, CD₃CN): δ [ppm] = 8.23 (s, 0.4H); 7.77 (s_{b}, 0.4H); 7.52 (d, 1H); 7.22-7.04 (m, 5H); 6.96 (t, 1H); 6.65-6.62 (m, 2H); 6.58 (d, 1H); 4.96 (t, 1H); 3.76 (s_{b}, 3H); 3.49-3.33 (m, 4H); 2.92-2.68 (m, 5H); 2.56-2.51 (m, 1H).

### Beispiel 28

### {2-[4-(3-Methoxyphenyl)-1-piperazinyl]-3-[2-methoxy-5-chlorphenyl]-3,4-dihydro-4-chinazolinyl}essigsäure

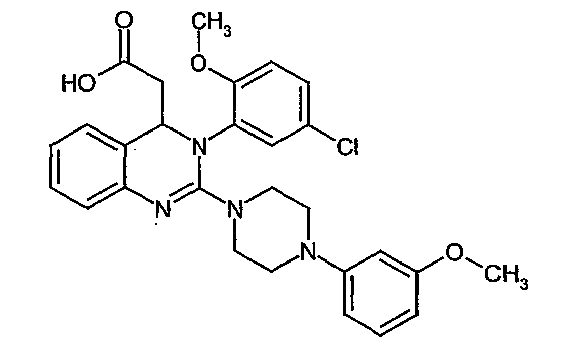

Ausgehend von 120 mg (0.2 mmol) Methylester aus Beispiel 18A werden nach der allgemeinen Arbeitsvorschrift [F] und Reinigung mittels präparativer HPLC 55 mg (47% d. Th.) Produkt erhalten.
HPLC (Methode 1): Rₜ = 4.40 min
MS (ESI-neg): m/z = 519 (M-H)⁻
¹H-NMR (400MHz, CD₃CN)- δ [ppm] = 8.21 (s, 0.5H); 7.41 (s_{b}, 0.5H); 7.22-7.16 (m, 3H); 7.11-7.06 (m, 2H); 6.98 (t, 1H); 6.95 (t, 1H); 6.71 (d, 1H); 6.41-6.37 (m, 2H); 6.34 (t, 1H); 4.93 (t, 1H); 3.71 (s, 3H); 3.66 (s_{b}, 3H); 3.48-3.42 (m, 4H); 2.97-2.75 (m, 5H); 2.54-2.48 (m, 1H).

### Beispiel 29

### {2-[4-(4-Fluoryphenyl)-1-piperazinyl]-3-[2-methoxy-5-chlorphenyl]-3,4-dihydro-4-chinazolinyl}essigsäure

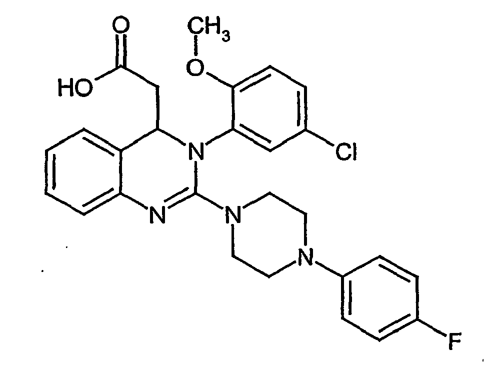

Ausgehend von 94 mg (0.18 mmol) Methylester aus Beispiel 19A werden nach der allgemeinen Arbeitsvorschrift [F] und Reinigung mittels präparativer HPLC 6 mg (7% d. Th.) Produkt erhalten.
HPLC (Methode 7): Rₜ=4.43 min
MS (ESI-pos): m/z = 509 (M+H)⁺
¹H-NMR (400MHz, CD₃CN): δ [ppm] = 8.11 (s, 0.5H); 7.23-7.09 (m, 4H); 7.13-6.96 (m, 6.5H); 6.87-6.82 (m, 2H); 4.84 (t, 1H); 3.72 (s, 3H); 3.48-3.42 (m, 4H); 2.93-2.87 (m, 2H); 2.83-2.77 (m, 1H); 2.47 (dd, 1H). Ein weiteres Proton vermutlich unter dem H₂O-Signal des Lösungsmittels (ca. 2.4-2. ppm).

### Beispiel 30

### {2-[4-(3-Methylphenyl)-1-piperazinyl]-3-[2-methoxy-5-methylphenyl]-3,4-dihydro-4-chinazolinyl}essigsäure

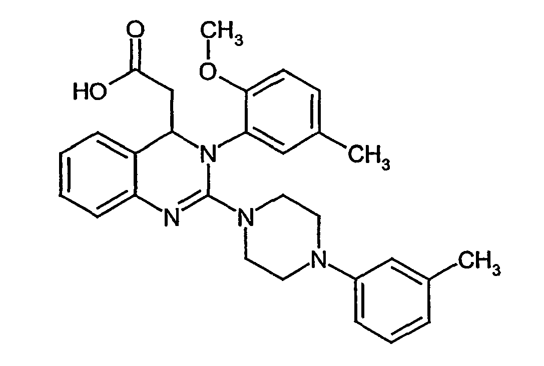

Ausgehend von 181 mg (0.36 mmol) Methylester aus Beispiel 20A werden nach der allgemeinen Arbeitsvorschrift [F] und Reinigung mittels präparativer HPLC 158 mg (86% d. Th.) Produkt erhalten.
HPLC (Methode 7): Rₜ= 4.63 min
MS (ESI-pos): m/z = 485 (M+H)⁺
¹H-NMR (400MHz, CD₃CN): δ [ppm] = 8.31 (s, 0.5H); 7.21-6.98 (m, 6H); 6.87-6.83 (m, 1H); 6.65-6.62 (m, 2H); 6.57 (d, 1H); 4.96-4.92 (m, 1H); 3.62 (s_{b}, 3H); 3.47-3.38 (m, 4H); 2.93-2.87 (m, 2H); 2.23 (s, 3H). Die Signale weiterer Protonen liegen vermutlich unter dem H₂O-Signal des Lösungsmittels (ca. 2.8-2.5 ppm).

### Beispiel 31

### {2-[4-(3-Chlorphenyl)-1-piperazinyl]-3-[2-methoxy-5-methylphenyl]-3,4-dihydro-4-chinazolinyl} essigsäure

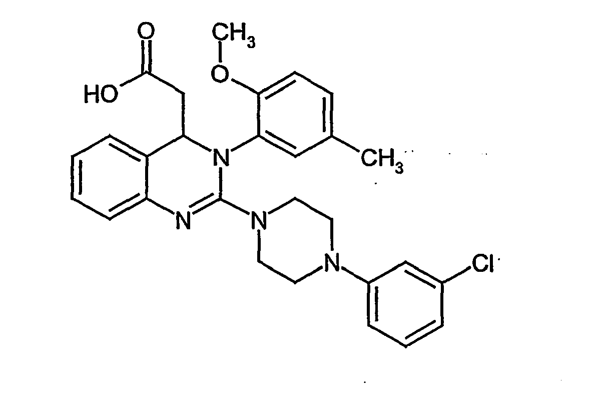

Ausgehend von 182 mg (0.36 mmol) Methylester aus Beispiel 21A werden nach der allgemeinen Arbeitsvorschrift [F] und Reinigung mittels präparativer HPLC 160 mg (88% d. Th.) Produkt erhalten.
HPLC (Methode 7): Rₜ=4.78 min
MS (ESI-pos): m/z = 505 (M+H)⁺
¹H-NMR (400MHz, CD₃CN): δ [ppm] = 8.27 (s, 0.5 H); 7.21-7.10 (m, 4.5H); 7.05-6.97 (m, 2H); 6.88-6.84 (m, 1H); 6.79-6.76 (m, 2H); 6.71 (d, 1H); 4.95-4.90 (m, 1H); 3.64 (s_{b}, 3H); 3.46-3.36 (m, 4H); 3.00-2.93 (m, 2H); 2.83-2.76 (m, 2H); 2.21 (s, 3H). Die Signale weiterer Protonen liegen vermutlich unter dem H₂O-Signal des Lösungsmittels (ca. 2.8-2.5 ppm).

### B. Bewertung der physiologischen Wirksamkeit

Die *in vitro*-Wirkung der erfindungsgemäßen Verbindungen kann in folgenden Assays gezeigt werden:

### Anti-HCMV- (Anti-Humanes Cytomegalo-Virus) Zytopathogenitätstests

Die Testverbindungen werden als 50 millimolare (mM) Lösungen in Dimethysulfoxid (DMSO) eingesetzt. Ganciclovir^{®}, Foscarnet^{®} und Cidofovir^{®} dienen als Referenzverbindungen. Nach der Zugabe von jeweils 2 µl der 50, 5, 0,5 und 0,05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0,001 - 0,002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0,0005 µM. Die Platten werden 6 Tage bei 37°C / 5 % CO₂ inkubiert, d.h. bis in den Viruskontrollen alle Zellen infiziert sind (100 % cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque Multiplier der Firma Technomara) visuell ausgewertet.

Die folgenden Daten können von den Testplatten ermittelt werden:
CC₅₀ (NHDF) = Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50 % im Vergleich zur unbehandelten Viruskontrolle hemmt;
SI (Selektivitätsindex) = CC₅₀ (NHDF) / EC₅₀ (HCMV).
Repräsentative in-vitro-Wirkdaten für die erfindungsgemäßen Verbindungen sind in Tabelle A wiedergegeben:

**Tabelle A**

| Beispiel N.r | NHDF CC₅₀ [uM] | HCMV EC₅₀ [uM] | SI HCMV |
|---|---|---|---|
| 1 | 17 | 0.027 | 630 |
| 2 | 39 | 0.06 | 650 |
| 6 | 22 | 0.4 | 63 |
| 19 | 24 | 0.4 | 60 |
| 23 | 188 | 0.76 | 247 |
| 26 | 31 | 0.019 | 1650 |
| 27 | 188 | 0.13 | 1446 |
| 28 | 63 | 0.025 | 2520 |
| 29 | 125 | 0.07 | 1786 |
| 30 | 250 | 0.25 | 1000 |
| 31 | 63 | 0.14 | 450 |

Die Eignung der erfindungsgemäßen Verbindungen zur Behandlung von HCMV-Infektionen kann im folgenden Tiermodell gezeigt werden:

### HCMV Xenograft-Gelfoam^{®}-Modell

### Tiere:

3-4 Wochen alte weibliche immundefiziente Mäuse (16-18 g), Fox Chase SCID oder Fox Chase SCID-NOD oder SCID-beige werden von kommerziellen Züchtern (Taconic M+B, Jackson, USA) bezogen. Die Tiere werden unter sterilen Bedingungen (einschließlich Streu und Futter) in Isolatoren gehalten.

### Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0,01-0,03 werden die virusinfizierten Zellen 5-10 Tage später geerntet und in Gegenwart von Minimal Essential Medium (MEM), 10 % foetalem Kälberserum (FKS) mit 10 % DMSO bei -40°C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot.

### Vorbereitung der Schwämme, Transplantation, Behandlung und Auswertung:

1x1x1 cm große Kollagenschwämme (Gelfoam^{®}; Fa. Peasel & Lorey, Best.-Nr. 407534; K.T. Chong et al., Abstracts of 39^{th} Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM + 10 % FKS aufbewahrt. 1 x 10⁶ virusinfizierte NHDF-Zellen (Infektion mit HCMV-Davis oder HCMV AD169 M.O.I = 0.03) werden 3 Stunden nach Infektion abgelöst und in 20 µl MEM, 10 % FKS auf einen feuchten Schwamm getropft. Ca. 16 Stunden später werden die infizierten Schwämme- mit 25 µl PBS / 0,1 % BSA / 1 mM DTT mit 5 ng/µl basic Fibroblast Growth Factor (bFGF) inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber verschlossen. 6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (14 Uhr) peroral mit Substanz behandelt. Die Tagesdosis beträgt beispielsweise 3 oder 10 oder 30 oder 60 oder 100 mg/kg Körpergewicht, das Applikationsvolumen 10 ml/kg Körpergewicht. Die Formulierung der Substanzen erfolgt in Form einer 0,5 %igen Tylosesuspension mit 2 % DMSO oder einer 0,5 %igen Tylosesuspension. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U/1,5 ml) aus dem Schwamm freigesetzt und in Gegenwart von MEM, 10 % foetalem Kälberserum, 10 % DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot. Ermittelt wird die Anzahl infektiöser Viruspartikel nach Substanzbehandlung im Vergleich zur placebobehandelten Kontrollgruppe.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5 %igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6 h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 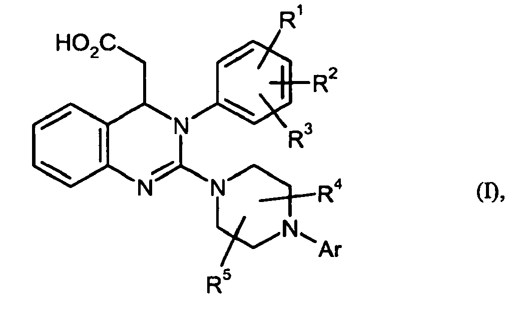 in welcher
Ar für C₆-C₁₄-Aryl steht, worin Aryl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Formyl, Carboxyl, C₁-C₆- Alkylcarbonyl, C₁-C₆-Alkoxycarbonyl, Trifluormethyl, Halogen, Cyano, Hydroxy, Amino, C₁-C₆-Alkylamino, Aminocarbonyl und Nitro,
worin Alkyl substituiert sein kann mit 1 bis 3 Substituenten, wobei die Substi- tuenten unabhängig voneinander ausgewählt werden aus der Gruppe, bestehend aus Halogen, Amino, C₁-C₆-Alkylamino, Hydroxy und C₆-C₁₄-Aryl,
oder zwei der Substituenten am Aryl zusammen mit den Kohlenstoffatomen, an die sie gebunden sind, ein 1,3-Dioxolan, einen Cyclopentan-Ring oder einen Cyclohexan-Ring bilden und ein gegebenenfalls vorhandener dritter Substituent unabhängig davon aus der genannten Gruppe ausgewählt wird,
R¹ für Wasserstoff, C₁-C₆-Alkyl, C₁-C₆-Alkoxy, Cyano, Halogen, Nitro oder Trifluormethyl steht,
R² für Wasserstoff, C₁-C₆-Alkyl, C₁-C₆-Alkoxy, Cyano, Halogen, Nitro oder Trifluormethyl steht,
R³ für C₁-C₆-Alkyl, C₁-C₆-Alkoxy, Cyano, Halogen, Nitro oder Trifluormethyl steht
oder einer der Reste R¹, R² und R³ für Wasserstoff, C₁-C₆-Alkyl, C₁-C₆-Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,
R⁴ für Wasserstoff oder C₁-C₆-Alkyl steht,
R⁵ für Wasserstoff oder C₁-C₆-Alkyl steht
oder
die Reste R⁴ und R⁵ im Piperazin-Ring an genau gegenüberliegenden Kohlenstoffatomen gebunden sind und eine gegebenenfalls mit 1 bis 2 Methylgruppen substituierte Methylen-Brücke bilden,
oder eines ihrer Salze, ihrer Solvate oder der Solvate ihrer Salze.

2. Verbindung nach Anspruch 1, **dadurch gekennzeichnet, dass**
Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
R¹ für Wasserstoff, Methyl, Methoxy, Fluor oder Chlor steht,
R² für Wasserstoff steht,
R³ für Methyl, iso-Propyl, tert.-Butyl, Cyano, Fluor, Chlor, Nitro oder Trifluormethyl steht,
R⁴ für Wasserstoff steht
und
R⁵ für Wasserstoff steht.

3. Verbindung nach Anspruch 1 oder 2, **dadurch gekennzeichnet, dass** R¹ für Wasserstoff, Methyl, Methoxy oder Fluor steht.

4. Verbindung nach Anspruch 3, **dadurch gekennzeichnet, dass** R¹ für Methyl oder Methoxy steht.

5. Verbindung nach einem der Ansprüche 1 bis 4, **dadurch gekennzeichnet, dass** R¹ über die ortho-Position zur Verknüpfungsstelle des Phenylrings an den Phenylring gebunden ist.

6. Verbindung nach einem der Ansprüche 1 und 3 bis 5, **dadurch gekennzeichnet, dass** R² für Wasserstoff steht.

7. Verbindung nach einem der Ansprüche 1 und 3 bis 6, **dadurch gekennzeichnet, dass** R³ für Trifluormethyl, Chlor, Methyl, iso-Propyl oder tert.-Butyl steht.

8. Verbindung nach einem der Ansprüche 1 bis 6, **dadurch gekennzeichnet, dass** R³ für Trifluormethyl, Chlor oder Methyl steht.

9. Verbindung nach einem der Ansprüche 1 bis 8, **dadurch gekennzeichnet, dass** R¹ über die ortho-Position zur Verknüpfungsstelle des Phenylrings an den Phenylring gebunden ist und R³ über die R¹ gegenüberliegende meta-Position zur Verknüpfungsstelle des Phenylrings an den Phenylring gebunden ist.

10. Verfahren zur Herstellung einer Verbindung der Formel (I) nach Anspruch 1, **dadurch gekennzeichnet, dass** eine Verbindung der Formel 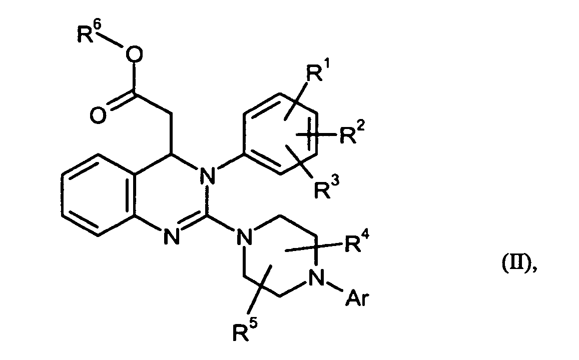 in welcher
r, R¹, R²,R³, R⁴ und R⁵ A die in Anspruch 1 angegebene Bedeutung haben und
R⁶ für Alkyl, bevorzugt Methyl oder Ethyl, steht,
mit einer Base umgesetzt wird.

11. Verbindung nach einem der Ansprüche 1 bis 9 zur Behandlung und/oder Prophylaxe von Krankheiten.

12. Verwendung einer Verbindung nach einem der Ansprüche 1 bis 9 zur Herstellung eines Arzneimittels zur Behandlung und/oder Prophylaxe von Krankheiten.

13. Verwendung einer Verbindung nach einem der Ansprüche 1 bis 9 zur Herstellung eines Arzneimittels zur Behandlung und/oder Prophylaxe von Virusinfektionen.

14. Verwendung nach Anspruch 13, **dadurch gekennzeichnet, dass** die Virusinfektion eine Infektion mit dem humanen Cytomegalovirus (HCMV) oder einem anderen Vertreter der Gruppe der Herpes viridae ist.

15. Arzneimittel enthaltend eine Verbindung nach einem der Ansprüche 1 bis 9 in Kombination mit einem weiteren Wirkstoff.

16. Arzneimittel nach Anspruch 15, **dadurch gekennzeichnet, dass** der weitere Wirkstoff ein antiviraler Wirkstoff ist.

17. Arzneimittel nach Anspruch 16, **dadurch gekennzeichnet, dass** der antivirale Wirkstoff Gancyclovir oder Acyclovir ist.

18. Arzneimittel enthaltend eine Verbindung nach einem der Ansprüche 1 bis 9 in Kombination mit einem inerten, nichttoxischen, pharmazeutisch geeigneten Hilfsstoff.

19. Arzneimittel nach Anspruch 15 zur Behandlung und/oder Prophylaxe von Virusinfektionen.

## Claims

1. Compound of the formula 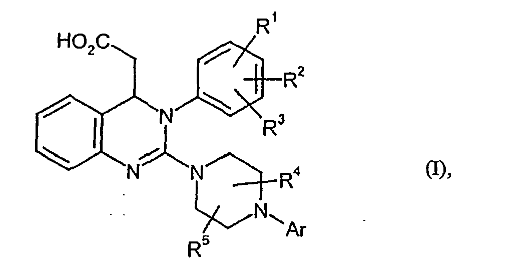 in which
Ar represents C₆-C₁₄-aryl which may be substituted by 1 to 3 substituents, where the substituents are selected independently of one another from the group consisting of C₁-C₆-alkyl, C₁-C₆-alkoxy, formyl, carboxyl, C₁-C₆-alkylcarbonyl, C₁-C₆-alkoxycarbonyl, trifluoromethyl, halogen, cyano, hydroxyl, amino, C₁-C₆-alkylamino, aminocarbonyl and nitro,
where alkyl may be substituted by 1 to 3 substituents, where the substituents are selected independently of one another from the group consisting of halogen, amino, C₁-C₆-alkylamino, hydroxyl and C₆- C₁₄-aryl,
or two of the substituents on the aryl radical together with the carbon atoms to which they are attached form a 1,3-dioxolane, a cyclo- pentane ring or a cyclohexane ring, and any third substituent present is selected independently therefrom from the group mentioned,
R¹ represents hydrogen, C₁-C₆-alkyl, C₁-C₆-alkoxy, cyano, halogen, nitro or trifluoromethyl,
R² represents hydrogen, C₁-C₆-alkyl, C₁-C₆-alkoxy, cyano, halogen, nitro or trifluoromethyl,
R³ represents C₁-C₆-alkyl, C₁-C₆-alkoxy, cyano, halogen, nitro or trifluoromethyl
or
one of the radicals R¹, R² and R³ represents hydrogen, C₁-C₆-alkyl, C₁-C₆-alkoxy, cyano, halogen, nitro or trifluoromethyl and the other two together with the carbon atoms to which they are attached form a 1,3-dioxolane, a cyclopentane ring or a cyclohexane ring,
R⁴ represents hydrogen or C₁-C₆-alkyl,
R⁵ represents hydrogen or C₁-C₆-alkyl,
or the radicals R⁴ and R⁵ are attached to carbon atoms directly opposing each other in the piperazine ring and form a methylene bridge which is optionally substituted by 1 or 2 methyl groups,
or a salt, a solvate or a solvate of a salt thereof.

2. Compound according to Claim 1, **characterized in that**
Ar represents phenyl which may be substituted by 1 or 2 substituents, where the substituents are selected independently of one another from the group consisting of methyl, methoxy, fluorine and chlorine,
R¹ represents hydrogen, methyl, methoxy, fluorine or chlorine, R² represents hydrogen,
R³ represents methyl, isopropyl, tert-butyl, cyano, fluorine, chlorine, nitro or trifluoromethyl,
R⁴ represents hydrogen
and
R⁵ represents hydrogen.

3. Compound according to Claim 1 or 2, **characterized in that** R¹ represents hydrogen, methyl, methoxy or fluorine.

4. Compound according to Claim 3, **characterized in that** R¹ represents methyl or methoxy.

5. Compound according to any of Claims 1 to 4, **characterized in that** R¹ is attached to the phenyl ring via the position ortho to the point of attachment of the phenyl ring.

6. Compound according to any of Claims 1 and 3 to 5, **characterized in that** R² represents hydrogen.

7. Compound according to any of Claims 1 and 3 to 6, **characterized in that** R³ represents trifluoromethyl, chlorine, methyl, isopropyl or tert-butyl.

8. Compound according to any of Claims 1 to 6, **characterized in that** R³ represents trifluoromethyl, chlorine or methyl.

9. Compound according to any of Claims 1 to 8, **characterized in that** R¹ is attached to the phenyl ring via the position ortho to the point of attachment of the phenyl ring and R³ is attached to the phenyl ring via the position meta to the point of attachment of the phenyl ring, which position is opposite to that of R¹.

10. Process for preparing a compound of the formula (I) according to Claim 1, **characterized in that** a compound of the formula 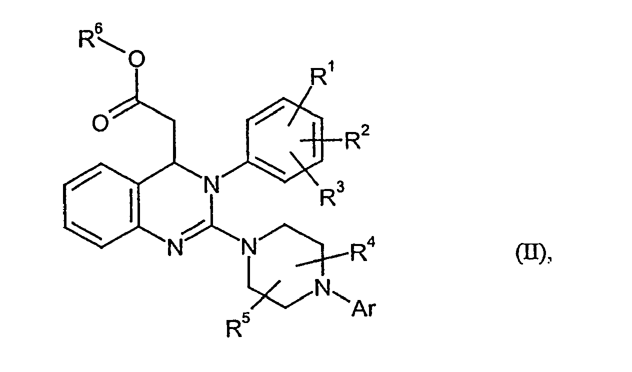 in which
Ar, R¹, R², R³, R⁴ and R⁵ are as defined in Claim 1 and
R⁶ represents alkyl, preferably methyl or ethyl,
is reacted with a base.

11. Compound according to any of Claims 1 to 9 for the treatment and/or the prophylaxis of diseases.

12. Use of a compound according to any of Claims 1 to 9 for preparing a medicament for the treatment and/or prophylaxis of diseases.

13. Use of a compound according to any of Claims 1 to 9 for preparing a medicament for the treatment and/or prophylaxis of viral infections.

14. Use according to Claim 13, **characterized in that** the viral infection is an infection with the human cytomegalovirus (HCMV) or another representative of the group of the Herpes viridae.

15. Medicament, comprising a compound according to any of Claims 1 to 9 in combination with a further active compound.

16. Medicament according to Claim 15, **characterized in that** the further active compound is an antiviral active compound.

17. Medicament according to Claim 16, **characterized in that** the antiviral active compound is gancyclovir or acyclovir.

18. Medicament, comprising a compound according to any of Claims 1 to 9 in combination with an inert non-toxic, pharmaceutically suitable auxiliary.

19. Medicament according to Claim 15 for the treatment and/or prophylaxis of viral infections.

## Revendications

1. Composé de formule 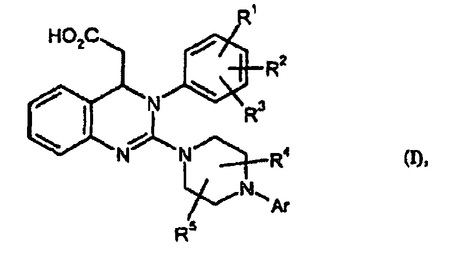 où
Ar représente C₆-C₁₄-aryle, où aryle peut être substitué par 1 à 3 substituants, les substituants étant choisis indépendamment l'un de l'autre dans le groupe constitué par C₁-C₆-alkyle, C₁-C₆-alcoxy, formyle, carboxyle, C₁-C₆-alkylcarbonyle, C₁-C₆- alcoxycarbonyle, trifluorométhyle, halogène, cyano, hydroxy, amino, C₁-C₆-alkylamino, aminocarbonyle et nitro,
où aryle peut être substitué par 1 à 3 substituants, les substituants étant choisis indépendamment l'un de l'autre dans le groupe constitué par halogène, amino, C₁-C₆-alkylamino, hydroxy et C₆-C₁₄-aryle,
ou deux des substituants sur aryle, ensemble avec les atomes de carbone auxquels ils sont liés, forment un 1,3-dioxolane, un cycle cyclopentane ou un cycle cyclohexane et un troisième substituant le cas échéant présent est choisi indépendamment de ceux-ci dans le groupe mentionné,
R¹ représente hydrogène, C₁-C₆-alkyle, C₁-C₆-alcoxy, cyano, halogène, nitro ou trifluorométhyle,
R² représente hydrogène, C₁-C₆-alkyle, C₁-C₆-alcoxy, cyano, halogène, nitro ou trifluorométhyle,
R³ représente C₁-C₆-alkyle, C₁-C₆-alcoxy, cyano, halogène, nitro ou trifluorométhyle
ou
un des radicaux R¹, R² et R³ représente hydrogène, C₁-C₆-alkyle, C₁-C₆-alcoxy, cyano, halogène, nitro ou trifluorométhyle et les deux autres, ensemble avec les atomes de carbone auxquels ils sont liés, forment un 1,3-dioxolane, un cycle cyclopentane ou un cycle cyclohexane,
R⁴ représente hydrogène ou C₁-C₆-alkyle,
R⁵ représente hydrogène ou C₁-C₆-alkyle,
ou
les radicaux R⁴ et R⁵ dans le cycle pipérazine sont liés à des atomes de carbone exactement opposés et forment un pont méthylène le cas échéant substitué par 1 à 2 groupes méthyle,
ou un de ses sels, ses solvates ou les solvates de ses sels.

2. Composé selon la revendication 1, **caractérisé en ce que**
Ar représente phényle, où phényle peut être substitué par 1 à 2 substituants, où les substituants sont choisis indépendamment l'un de l'autre dans le groupe constitué par méthyle, méthoxy, fluor et chlore,
R¹ représente hydrogène, méthyle, méthoxy, fluor ou chlore,
R² représente hydrogène,
R³ représente méthyle, iso-propyle, tert-butyle, cyano, fluor, chlore, nitro ou trifluorométhyle,
R⁴ représente hydrogène
et
R⁵ représente hydrogène.

3. Composé selon la revendication 1 ou 2, **caractérisé en ce que** R¹ représente hydrogène, méthyle, méthoxy ou fluor.

4. Composé selon la revendication 3, **caractérisé en ce que** R¹ représente méthyle ou méthoxy.

5. Procédé selon l'une quelconque des revendications 1 à 4, **caractérisé en ce que** R¹ est lié sur le cycle phényle en position ortho par rapport au site de liaison du cycle phényle.

6. Composé selon l'une quelconque des revendications 1 et 3 à 5, **caractérisé en ce que** R² représente hydrogène.

7. Composé selon l'une quelconque des revendications 1 et 3 à 6, **caractérisé en ce que** R³ représente trifluorométhyle, chlore, méthyle, iso-propyle ou tert-butyle.

8. Composé selon l'une quelconque des revendications 1 à 6, **caractérisé en ce que** R³ représente trifluorométhyle, chlore ou méthyle.

9. Composé selon l'une quelconque des revendications 1 à 8, **caractérisé en ce que** R¹ est lié au cycle phényle en position ortho par rapport au site de liaison du cycle phényle et R³ est lié au cycle phényle en position méta opposée à R¹ par rapport au site de liaison du cycle phényle.

10. Procédé pour la préparation d'un composé de formule (I) selon la revendication 1, **caractérisé en ce qu'**on transforme un composé de formule 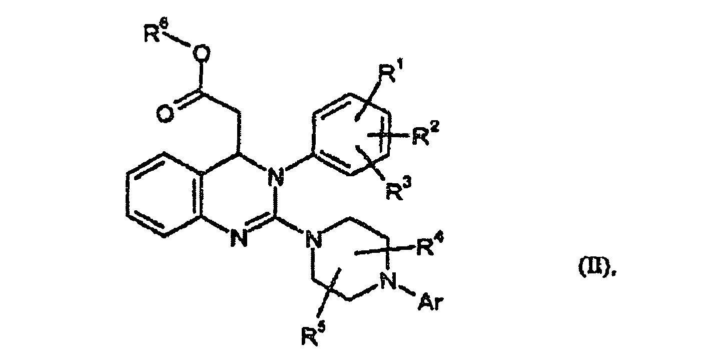 où
Ar, R¹, R², R³, R⁴ et R⁵ ont la signification indiquée dans la revendication 1 et
R⁶ représente alkyle, de préférence méthyle ou éthyle,
avec une base.

11. Composé selon l'une quelconque des revendications 1 à 9 destiné au traitement et/ou à la prophylaxie de maladies.

12. Utilisation d'un composé selon l'une quelconque des revendications 1 à 9 pour la préparation d'un médicament destiné au traitement et/ou à la prophylaxie de maladies.

13. Utilisation d'un composé selon l'une quelconque des revendications 1 à 9 pour la préparation d'un médicament destiné au traitement et/ou à la prophylaxie d'infections virales.

14. Utilisation selon la revendication 13, **caractérisée en ce que** l'infection virale est une infection par le cytomégalovirus humain (HCMV) ou un autre représentant du groupe des Herpes viridae.

15. Médicament contenant un composé selon l'une quelconque des revendications 1 à 9 en combinaison avec une autre substance active.

16. Médicament selon la revendication 15, **caractérisé en ce que** l'autre substance active est une substance active antivirale.

17. Médicament selon la revendication 16, **caractérisé en ce que** la substance active antivirale est le Gancyclovir ou l'Acyclovir.

18. Médicament contenant un composé selon l'une quelconque des revendications 1 à 9 en combinaison avec un adjuvant inerte, non toxique, pharmaceutiquement approprié.

19. Médicament selon la revendication 15 destiné au traitement et/ou à la prophylaxie d'infections virales.
